(19) Europäisches Patentamt
European Patent Office
Office européen des brevets

(11) EP 1 379 675 B1

(12) # EUROPÄISCHE PATENTSCHRIFT

(45) Veröffentlichungstag und Bekanntmachung des Hinweises auf die Patenterteilung:
**05.11.2008 Patentblatt 2008/45**

(21) Anmeldenummer: **02759795.4**

(22) Anmeldetag: **05.04.2002**

(51) Int Cl.:
***C12P 7/24*** *(2006.01)* ***C12P 7/22*** *(2006.01)*
***C12P 7/40*** *(2006.01)*

(86) Internationale Anmeldenummer:
**PCT/EP2002/003803**

(87) Internationale Veröffentlichungsnummer:
**WO 2002/081718 (17.10.2002 Gazette 2002/42)**

(54) **VERFAHREN ZUR OXIDATION AROMATISCHER VERBINDUNGEN**

METHOD FOR THE OXIDATION OF AROMATIC COMPOUNDS

PROCEDE D'OXYDATION DE COMPOSES AROMATIQUES

(84) Benannte Vertragsstaaten:
**AT BE CH CY DE DK ES FI FR GB GR IE IT LI LU MC NL PT SE TR**

(30) Priorität: **06.04.2001 DE 10117359**

(43) Veröffentlichungstag der Anmeldung:
**14.01.2004 Patentblatt 2004/03**

(73) Patentinhaber: **BASF SE**
**67056 Ludwigshafen (DE)**

(72) Erfinder:
- **HAUER, Bernhard**
**67136 Fussgönheim (DE)**
- **ZELINSKI, Thomas**
**67122 Altrip (DE)**
- **WITHOLT, Bernhard**
**8032 Zürich (CH)**
- **SCHMID, Andreas**
**CH-8049 Zürich (CH)**
- **BÜHLER, Bruno**
**CH-8048 Zürich (CH)**

(74) Vertreter: **Kinzebach, Werner**
**Reitstötter, Kinzebach & Partner**
**Patentanwälte**
**Sternwartstrasse 4**
**81679 München (DE)**

(56) Entgegenhaltungen:
- **WUBBOLTS M G ET AL: "BIOSYNTHESIS OF SYNTHONS IN TWO-LIQUID-PHASE MEDIA" BIOTECHNOLOGY AND BIOENGINEERING, INTERSCIENCE PUBLISHERS, LONDON, GB, Bd. 52, Nr. 2, 20. Oktober 1996 (1996-10-20), Seiten 301-308, XP000972414 ISSN: 0006-3592**
- **WUBBOLTS M G ET AL: "Efficient production of optically active styrene epoxides in two-liquid phase cultures." ENZYME AND MICROBIAL TECHNOLOGY, Bd. 16, Nr. 10, 1994, Seiten 887-894, XP001086459 ISSN: 0141-0229**
- **PANKE SVEN ET AL: "Production of enantiopure styrene oxide by recombinant Escherichia coli synthesizing a two-component styrene monooxygenase." BIOTECHNOLOGY AND BIOENGINEERING, Bd. 69, Nr. 1, 5. Juli 2000 (2000-07-05), Seiten 91-100, XP002206959 ISSN: 0006-3592**
- **BUHLER BRUNO ET AL: "Xylene monooxygenase catalyzes the multistep oxygenation of toluene and pseudocumene to corresponding alcohols, aldehydes, and acids in Escherichia coli JM101." JOURNAL OF BIOLOGICAL CHEMISTRY, Bd. 275, Nr. 14, 7. April 2000 (2000-04-07), Seiten 10085-10092, XP002206960 ISSN: 0021-9258 in der Anmeldung erwähnt**
- **PANKE SVEN ET AL: "Engineering of a stable whole-cell biocatalyst capable of (S)-styrene oxide formation for continuous two-liquid-phase applications." APPLIED AND ENVIRONMENTAL MICROBIOLOGY, Bd. 65, Nr. 12, Dezember 1999 (1999-12), Seiten 5619-5623, XP002206961 ISSN: 0099-2240**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

• SCHMID A ET AL: "INDUSTRIAL BIOCATALYSIS TODAY AND TOMORROW" NATURE, MACMILLAN JOURNALS LTD. LONDON, GB, Bd. 409, 11. Januar 2001 (2001-01-11), Seiten 258-268, XP000985347 ISSN: 0028-0836

## Beschreibung

Die Erfindung betrifft ein Verfahren zur biokatalytischen Oxidation aromatischer Verbindungen.

Aromatischen Verbindungen, wie Aldehyde, sind wichtige Inhaltsstoffe in Geruchs- und Aromastoffen (Wittcoff und Reuben 1996). Ebenso gelten sie als Ausgangsstoffe für verschiedene Polymere, Pharmazeutikas und Feinchemikalien.

Der wichtigste chemische Syntheseweg zur Herstellung eines typischen aromatischen Aldehyds, nämlich des Benzaldehyds, erfolgt ausgehend von Toluol (Wittcoff und Reuben 1996). Durch Dichlorierung von Toluol entsteht Benzylidenchlorid, welches durch Hydrolyse in Benzaldehyd umgeformt wird (Schema 1).

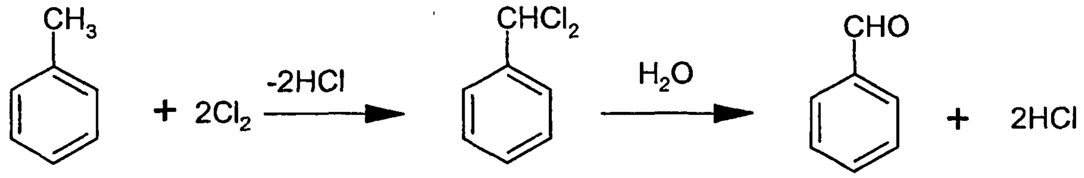


Substrat: Toluol

Benzylidenchlorid

Benzaldehyd

Schema 1: Herstellung von Benzaldehyd aus Toluol

Die spezifische Oxidation nur einer Methylgruppe oder die Aufreinigung aus Produktgemischen stellt bei chemischen Synthesen die grösste Schwierigkeit dar. Ausserdem müssen die Reaktionen oftmals mit Hilfe von äusserst giftigen Substanzen oder unter extremen Reaktionsbedingungen durchgeführt werden. Des weiteren bereiten die bei der chemischen Synthese gebildeten Nebenprodukte Probleme. Aus den oben aufgeführten Gründen ist z. B. die Herstellung von 3,4-Dimethylbenzaldehyd mittels chemischer Synthese sehr schwierig.

Die biokatalytische Herstellung von aromatischen Verbindungen, wie Aldehyden, stellt eine Alternative zur oben beschriebenen chemischen Herstellung dar.

Für die Produktion von aromatischen Aldehyden werden hauptsächlichdie Enzymklassen Transferasen, Lyasen (Simmonds and Robinson 1997; Simmonds and Robinson 1998) und Oxidoreduktasen (Legoy, Kim et al. 1985; Molinari, Villa et al. 1995; Molinari, Aragozzini et al. 1997; Molinari, Gandolfi et al. 1999) verwendet. Die Oxidoreduktasen sind von speziellen Interesse, da sie in der Lage sind stereo- und regioselektiv nur eine Methylgruppe, z. B. von Xylolen, zu oxidieren. Xylole gelten als kostengünstige Substrate und können in einer Zweischrittreaktion in die entsprechenden Aldehyde umgeformt werden.

Das TOL-Plasmid pWWO von *Pseudomonas putida* mt-2 ermöglicht den Katabolismus von Toluolen und Xylolen. Auf dem Plasmid sind der obere und der *meta*-cleavage Abbauweg auf zwei separaten Operons kodiert. Die Enzyme des oberen Abbauweges katalysieren die Oxidation von Toluolen oder Xylolen zu den entsprechenden Benzoesäuren und die Enzyme des meta-cleavage Abbauwegs diejenigen Reaktionen, die Benzoesäure zu Substraten des Zitronensäurezyklus abbauen (Harayama, Rekik et al. 1989; Harayama, Kok et al. 1992; Ramos, Marqués et al. 1997; Williams, Shaw et al. 1997). Der obere Abbauweg ist für die Herstellung von aromatischen Aldehyden von besonderem Interesse. Das Operon des oberen Abbauweges enthält 5 Gene (Harayama, Leppik et al. 1986). Wie in Schema 2 ersichtlich, ist das erste Enzym die Xylolmonooxygenase (XMO), welches Toluole oder Xylole in die entsprechenden Benzylalkohole umwandelt.

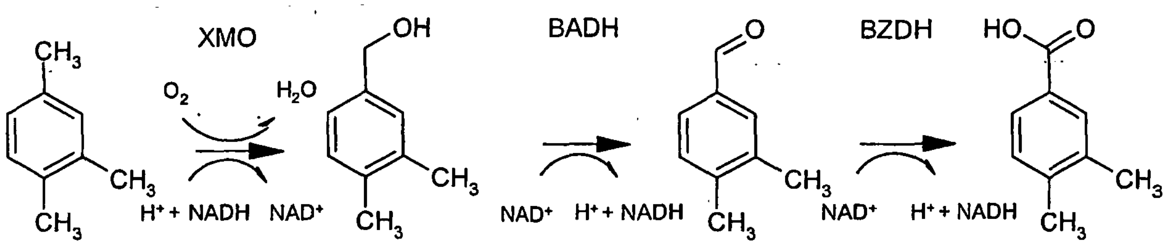


Schema 2: Oberer Abbauweg von *Pseudomonas putida*-mt2

XMO: Xylolmonooxygenase
BADH: Benzylalkoholdehydrogenase
BZDH: Benzaldehyddehydrogenase

Das zweite Enzym ist die Benzylalkoholdehydrogenase (BADH), welche fähig ist die Reaktion von Benzylalkoholen zu Benzaldehyden zu katalysieren. Bei den beschriebenen Reaktionen wird von der Xylolmonooxygenase NADH verbraucht und von der Alkoholdehydrogenase wieder gebildet. Dies ergäbe beim Einsatz dieser beiden Enzyme zur Produktion von Benzaldehyden eine ausgeglichene NADH - Bilanz.

Bei gemeinsamer Verwendung von XMO und BADH in rekombinanten *E.coli* zur Umsetzung von Toluolen zu den entsprechenden Benzaldehyden, wurden die Benzaldehyde nur in geringem Mass gebildet. Es wurde vielmehr eine Rückbildung der Benzaldehyde in die entsprechenden Benzylalkohole beobachtet (Bühler, Schmid et al. 2000). Es konnte gezeigt werden, dass das Gleichgewicht der durch die BADH katalysierten Reaktionen stark auf der Seite der Alkohole liegt.

Nach Bühler, Schmid et al. (2000) ist die Xylolmonooxygenase, in Abwesenheit der BADH aber fähig, die Mehrstufenoxidation von einer Methylgruppe von Toluolen oder Xylolen in Benzylalkohol, Benzaldehyd und Benzoesäure alleine zu katalysieren. Dies geschieht durch die Einführung eines Sauerstoffatoms von molekularem Sauerstoff, also über Monooxygenierungen (Bühler, Schmid et al. 2000). In Schema 3 sind die durch die XMO katalysierten Reaktionen dargestellt. Dabei ist zu beachten, dass es sich bei den Monooxygenierungen im Gegensatz zu den Dehydrogenierungen um irreversible Reaktionen handelt.

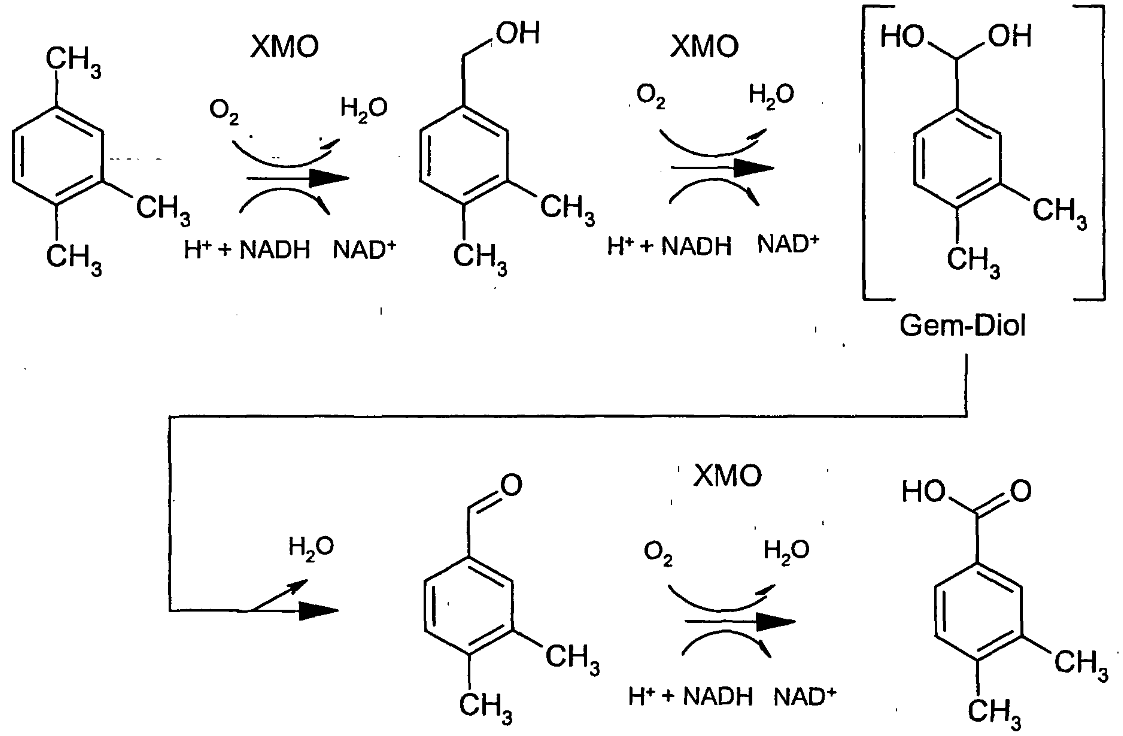


Schema 3: Durch die Xylolmonooxygenase (XMO) katalysierte Reaktionswege

Zur Steuerung der Expression von XMO in rekombinanten *E. coli* wurde das alk Regulationssystem verwendet, welches durch n-Octan induziert werden kann (Panke, Meyer et al. 1999) (siehe Figur 1).

Die biotechnologische Herstellung von selektiv oxidierten Aromaten, wie 3,4-Dimethylbenzaldehyd, aus billigem Substrat ist von grossem Interesse, da eine regiospezifische Oxidation von nur einer Methylgruppe mittels chemischer Synthese sehr schwierig ist. Dabei sind die niederen Wasserlöslichkeiten und die Toxizitäten der Substrate und der Produkte für die Zellen von entscheidender Bedeutung. So haben Toluole schon bei einer Konzentration von 1 - 4 mM eine toxische Wirkung auf E. *coli (Schmid 1997).* Dies limitiert die Anwendbarkeit des wässrigen Einphasensystems.

In der älteren Deutschen Patentanmeldung DE-A-199 51 768.1 wird ein Verfahren zur biokatalytischen Herstellung aromatischer Aldehyde und/oder Carbonsäuren in einphasigem Reaktionsmedium unter Verwendung XMO-exprimierender Mikroorganismen beschrieben.

## Kurze Beschreibung der Erfindung:

Aufgabe der vorliegenden Erfindung ist die Bereitstellung eines verbesserten biokatalytischen Verfahrens zur Oxidation von aromatischen Verbindungen mit Hilfe von XMOproduzierenden Mikroorganismen. Insbesondere sollte das neue Verfahren für die Durchführung in größerem, halbtechnischen oder technischen, Maßstab geeignet sein.

Überraschenderweise konnte anhand des Modellsystems der mikrobiologischen Oxidation von Pseudocumol zu den entsprechenden Oxidationsprodukten (Alkohol, Aldehyd, Coarbonsäure) gezeigt werden, dass obige Aufgabe durch Einsatz eines zweiphasigen wässrig/organischen Reaktionsmediums gelöst werden kann. Das erfindungsgemäße Zweiphasensystem zeichnet sich gegenüber einem herkömmlichen wässrigen Einphasensystem durch einige gravierende Vorteile aus (Tabelle 1).

Tabelle 1: Vorteile von Mehrphasen-Systemen

- erhöhte Konzentrationen von gering wasserlöslichen Substraten / Produkten;
- erleichterte Produktaufarbeitung durch Phasentrennung;
- Verringerung von Substrat- bzw. Produktinhibierungen;
- Verschiebung des Reaktionsgleichgewichtes zu höheren Umsätzen durch Produktextraktion;

- Reversion einer hydrolytischen Reaktion durch Verringerung der Wasseraktivität;
- kleine Reaktorvolumina und Volumenströme
- geringe Gefahr mikrobieller Kontaminationen
- geringe Gefahr der hydrolytischen Spaltung von labilen Substraten bzw. Produkten

Detaillierte Beschreibung der Erfindung:

Die vorliegende Erfindung betrifft insbesondere ein Verfahren zur ein- oder mehrstufigen biokatalytischen Oxidation aromatischer Verbindungen, das dadurch gekennzeichnet, dass man

a) in einem zweiphasigen, wässrig-organischen Kulturmedium, welches ein aromatisches Substrat der Formel II

$$Ar\text{-}R^2 \qquad (II)$$

worin
Ar für einen gegebenenfalls ein- oder mehrfach substituierten einkernigen aromatischen Ring steht; und
$R^2$ für $-(CH_2)_{n+1}R^3$ steht, worin n für eine ganzzahligen Wert von 0 bis 15, wie z. B. 0, 1, 2, 3, 4 oder 5 steht; und
$R^3$ für H steht;
enthält, einen Mikroorganismus unter aeroben Bedingungen kultiviert, der ein Enzym mit Xylolmonooxygenase (XMO)-Aktivität exprimiert; und

b) wenigstens ein bei der XMO-katalysierten Oxidationsreaktion gebildetes Produkt, worin der Rest $R^2$ zum korrespondierenden Hydroxy-, Carbonyl- oder Carboxyl-Rest oxidiert wurde, aus dem Kulturmedium isoliert; und wobei der Oxidationsgrad des eingesetzten Substrats über die Einstellung der Substrat Konzentration steuerbar ist.

a) Eingesetzte Edukte:

Die erfindungsgemäßen Reaktionen können mit dem gleichen Enzym (XMO), ein- oder mehrstufig durchgeführt werden. Als Substrat wird der alkylierte Aromat eingesetzt. Der gewünschte Oxidationsgrad des eingesetzten Substrats kann, wie später noch genauer erläutert, gesteuert werden.

Das aromatische Ringsystem Ar in der erfindungsgemäß hergestellten bzw. als Substrat eingesetzten Verbindungen der Formeln I und II kann einfach oder mehrfach substituiert sein. Die Position des/der Ringsubstituenten ist beliebig wählbar. Bevorzugt ist jedoch die meta- und/oder para-Stellung zur zu oxidierenden Seitenkette.

Geeignete Substituenten am Aromaten umfassen $C_1$-$C_4$-Alkyl, wie Methyl, Ethyl, n- und i-Propyl und n-Butyl, insbesondere Methyl und Ethyl, $C_1$-$C_4$-Alkoxy, insbesondere Methoxy und Ethoxy, Halogen, wie F, Cl, Br und I, insbesondere Cl, und Nitro.

Konkrete nichtlimitierende Beispiele für nach dem erfindungsgemäßen Verfahren durch XMO oxidierbare Substrate der Formel II sind Toluol, Xylole, m- und/oder p- Methyl, Ethyl-, Methoxy-, Nitro- und Chlor-substituierte Toluole, sowie m-Bromsubstituiertes Toluol und Pseudocumol (d.h. Trimethylbenzole).

b) Organische Phase

Die Wahl der organischen Phase ist für das erfindungsgemäß verwendete Zweiphasensystem von Bedeutung und hängt von verschiedenen Parametern ab:

- die toxische oder inhibierende Wirkung der organischen Phase auf die Zellen
- die Löslichkeit der Substrate und Produkte in der organischen Phase
- die Verteilungskoeffizienten von Substrat und Produkt
- Entflammbarkeit und Giftigkeit der organischen Phase
- Dichteunterschied der organischen Phase zu Wasser
- der Siedepunkt der organischen Phase

Erfindungsgemäß bevorzugt verwendet man für die organische Phase des zweiphasigen Reaktionsmediums eine apolare organische Verbindung, die in einem n-Octanol/Wasser-Zweiphasensystem einen Verteilungskoeffizienten von $>10^4$ aufweist.

Außerdem ist die Verwendung solcher organischer Phasen bevorzugt, die bei 1 atm eine Siedepunkt oder Siedebereich aufweist, der um etwa 50 bis 200 °C über dem Siedepunkt des Oxidationsprodukts (der Oxidationsprodukte), insbesondere des am höchsten siedenden zu isolieren gewünschten Oxidationsprodukts, liegt. Besonders bevorzugt verwendet man als organische Phase Di-($C_5$-$C_{12}$-alkyl)-phthalat oder ein Gemisch solcher Phthalate. Besonders bevorzugt verwendet man Dioctylphthalat (Bisethylhexylphthalat) und solche die gleichzeitig als Antischaummittel während der Fermentation fungieren können.

Nach einer bevorzugten Variante des erfindungsgemäßen Verfahrens gewinnt man das Reaktionsprodukt dadurch, dass man die organische Phase abtrennt und das (die) darin gelöste(n) Oxidationsprodukt(e) abdestilliert.

c) Eingesetzter Biokatalysator und Expressionskonstrukte

Die erfindungsgemäßen Verfahren werden bevorzugt unter Einsatz von XMO, kodiert von den Genen xylA und xylB gemäß xylMA GENBANK-Accession Nr. M37480 und korrespondierenden Isoenzymen durchgeführt. XMO stammt bevorzugt aus Bakterien der Gattung Pseudomonas, insbesondere der Spezies Pseudomonas putida, bevorzugt Stamm mt-2 (ATCC 33015).

Erfindungsgemäß mit umfasst ist ebenfalls die Verwendung "funktionaler Äquivalente" der konkret offenbarten XMO's.

"Funktionale Äquivalente" oder Analoga der konkret offenbarten Monooxygenasen sind im Rahmen der vorliegenden Erfindung davon verschiedene Enzyme, welche weiterhin die gewünschte Reaktion zeigen und zur Herstellung von Alkoholen, Aldehyden und/oder Carbonsäuren obiger allgemeiner Formel I brauchbar sind.

Unter "funktionalen Äquivalenten" versteht man erfindungsgemäß insbesondere Enzymmutanten, welche in wenigstens einer Sequenzposition eine andere als die ursprüngliche Aminosäure aufweisen aber trotzdem eine der oben genannten Oxidationsreaktionen katalysieren. "Funktionale Äquivalente" umfassen somit die durch eine oder mehrere Aminosäure-Additionen, -Substituenten, -Deletionen und/oder -Inversionen erhältlichen Mutanten, wobei die genannten Veränderungen in jeglicher Sequenzposition auftreten können, solange sie zu einer Mutante mit dem erfindungsgemäßen katalytischen Aktivität führen. Funktionale Äquivalenz ist insbesondere auch dann gegeben, wenn die Reaktivitätsmuster zwischen Mutante und unverändertem Enzym qualitativ übereinstimmen, d.h. beispielsweise gleiche Substrate mit unterschiedlicher Geschwindigkeit umgesetzt werden.

"Funktionale Äquivalente" umfassen natürlich auch Monooxygenasen, welche aus anderen Organismen, z.B. aus anderen als den hierin konkret genannten Bakterien, zugänglich sind, sowie natürlich vorkommende Varianten oder Isoezyme. Beispielsweise lassen sich durch Sequenzvergleich Bereiche homologer Sequenzregionen festlegen und in Anlehnung an die konkreten Vorgaben der Erfindung äquivalente Enzyme ermitteln.

Erfindungsgemäß mit umfaßt ist auch die Verwendung von anderen als den konkret genannten Nukleinsäuresequenzen (einzel- und doppelsträngige DNA- und RNA-Sequenzen) welche für eine der obigen Monooxygenasen und deren funktionalen Äquivalenten kodieren. Weitere erfindungsgemäß brauchbare Nukleinsäuresequenzen unterscheiden sich somit von den konkret eingesetzten Sequenzen durch Addition, Substitution, Insertion oder Deletion einzelner oder mehrerer Nukleotide, kodieren aber weiterhin für eine Monooxygenase mit der gewünschten Eigenschaftsprofil.

Erfindungsgemäß umfasst ist auch der Einsatz solcher Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Die kodierende XMO Sequenz ist Bestandteil von Expressionskonstrukten, enthaltend unter der genetischen Kontrolle regulativer Nukleinsäuresequenzen eine für ein erfindungsgemäß brauchbares Monooxygenase-Enzym kodierende Nukleinsäuresequenz. Vorzugsweise umfassen solche erfindungsgemäßen Konstrukte 5'-strom-aufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Translationsverstärker, Enhancer, Polyadenylierungssignale und dergleichen. Weitere regulative Elemente umfassen selektierbare Marker, Amplifikationssignale, Replikationsursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird.

Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, lpp-lac-, laclq-, T7-, T5-, T3-, gal-, trc-, ara-, SP6-, l-PR- oder l-PL-Promotor, die vorteilhafterweise in gram-negativen Bakterien Anwendung finden; sowie die gram-positiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS1, B33, not oder der Ubiquitin- oder Phaseolin-Promotor. Besonders bevorzugt ist die Verwendung induzierbarer Promotoren, wie z.B. licht- oder temperaturinduztierbare Promotoren, wie der $P_rP_l$-Promotor

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer erfindungsgemäßen Expressionskassette erfolgt durch Fusion eines geeigneten Promotors mit einer geeigneten Monooxygenase-Nukleotidsequenz sowie einem Terminator- oder Polyadenylierungssignal. Dazu verwendet man gängige Rekombinations- und Klonierungstechniken, wie sie beispielsweise in Sambrook et al beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al.) entnommen werden.

Unter Vektoren sind außer Plasmiden auch alle anderen dem Fachmann bekannten Vektoren, wie beispielsweise Phagen, Viren, wie SV40, CMV, CaMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Mit Hilfe solcher erfindungsgemäßer Vektoren sind rekombinante Mikroorganismen herstellbar, welche beispielsweise mit wenigstens einem erfindungsgemäßen Vektortransformiert sind und im erfindungsgemäßen Verfahren eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al. beschrieben.

d) Mikroorganismen

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen und zur Durchführung der erfindungsgemäßen mikrobiologischen Oxidationsreaktion einsetzbar sind. Unter Wirtsorganismen sind beispielsweise Bakterien, Pilze, Hefen, pflanzliche oder tierische Zellen zu verstehen. Bevorzugt Organismen sind Bakterien.

Bevorzugt wird jedoch ein solcher XMO exprimierender Mikroorganismus verwendet, der im wesentlichen keine Benzylalkoholdehydrogenase (BADH) und/oder keine Benzaldehyddehydrogenase (BZDH)-Aktivität besitzt. Insbesondere bevorzugt verwendet man einen Mikroorganismus der mit dem Expressionsplasmid pSPZ3 transformiert ist.

Die zur Durchführung des Verfahrens vorzugsweise verwendeten Mikroorganismus sind Bakterien der Gattung Escherichia, insbesondere E. coli, wie z.B. der Stamm JM101.

Die Transformation von Mikroorganismen mit einem Vektor erfolgt erfindungsgemäß nach etablierten Standardtechniken und bedarf daher keiner detaillierteren Erörterung.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Die Kombination aus den Wirtsorganismen und den zu den Organismen passenden Vektoren, wie Plasmide,

Viren oder Phagen, wie beispielsweise Plasmide mit dem RNA-Polymerase/Promoter-System, die Phagen λ oder μ oder andere temperente Phagen oder Transposons und/oder weiteren vorteilhaften regulatorischen Sequenzen bildet ein Expressionssystem.

Gemäß einer besonders bevorzugten Ausführungsform verwendet man einen rekombinanten Mikroorganismus, welcher mit einem Expressionsvektor transformiert ist, der, z.B. unter der genetischen Kontrolle des alk-Regulationssystems aus Pseudomonas oleovorans GPo1, die für XMO kodierenden Gene xylM und xylA trägt.

Insbesondere bevorzugt ist der Mikroorganismus mit dem xylMA kodierenden Expressionsplasmid pSPZ3 transformiert.

Das alk-Regulationssystem aus Pseudomonas oleovorans GPo1 ist an sich bekannt. Die Expression des ersten der zwei oben erwähnen alk-Gencluster steht unter der Kontrolle von alkBp, dem alk-Promotor, und beginnt in Gegenwart des funktionellen Regulationsproteins alkS, das von dem zweiten alk-Gencluster kodiert wird, und in Gegenwart eines Induktors, wie z. B. einem Alkan, beispielsweise n-Octan, oder einer mit diesen wenig verwandten Verbindung, wie z. B. Dicyclopropylketon (DCPK) (8, 22, 23). Die Verwendung des alk-Regulationssystems in E. coli hat den Vorteil, dass keine Katabolitrepression eintritt.

e) Durchführung des Verfahrens

Das erfindungsgemäße Verfahren kann diskontinuierlich, halbkontinuierlich oder kontinuierlich in herkömmlichen Bioreaktoren durchgeführt werden. Besonders bevorzugt ist die Anwendung einer semikontinuierlichen Batchfahrweise. Dabei wird nach Beendigung der Umsetzung das Kulturmedium bis auf einen kleinen Rest, wie z.B. etwa 1 bis 5 Vol-% aus dem Reaktor entfernt. Dieser Rest dient dann als Impfkultur für das folgende Batch.

Die Wahl der optimalen Verfahrensparameterkonzentration, wie Belüftungrate und Sauerstoffeintrag, Eduktkonzentration, pH-Wert, Temperatur, Zusammensetzung des Reaktionsmediums bezüglich organischer Phase und Nährmedium, Zulaufdauer und - geschwindigkeit und dergleichen können vom Fachmann unter Berücksichtigung der Offenbarung im folgenden experimentellen Teil ohne unzumutbarem Aufwand vorgenommen werden. So kann beispielsweise der pH-Wert des Reaktionsmediums als Regelgröße für die Produktivität des Biokatalysators verwendet werden.

In dem erfindungsgemäßen Verfahren wird bevorzugt n-Octan als Induktor verwendet, und zwar besonders bevorzugt in einer Menge von 0,001 bis 0,5% (v/v)

In einer weiteren bevorzugten Verfahrensvariante wird die erfindungsgemäße Umsetzung in im wesentlichen Antibiotika-freien Reaktionsmedien durchgeführt. Dies ist überraschend vorteilhaft, da unter den herrschenden Reaktionsbedingungen eigentlich zu erwarten gewesen wäre, dass der verwendete rekombinante Mikroorganismus das eingeführte Plasmid verliert. Der erfindungsgemäß ermöglichte Verzicht auf Antibiotika im Reaktionsmedium bedeutet eine signifikante Kostenersparnis, da auf den Antibiotikaeinsatz verzichtet werden kann und außerdem keine Antibiotikarückstande aus dem aufgearbeiteten Reaktionsmedium entfernt werden müssen.

Der Oxidationsgrad der erfindungsgemäß eingesetzten Substrate kann auf unterschiedliche Weise gesteuert werden. Beispielsweise werden in regelmäßigen Abständen Proben aus dem Kulturmedium entnommen und gaschromatographisch oder unter Anwendung der Gaschromatographie-Massenspektrometer-Kopplung (GC-MS) oder der Hochleistungsflüssigkeitschromatographie auf den Gehalt an den entsprechenden Alkohol-, Aldehyd- und/oder Carbonsäure-Derivaten untersucht. Je nachdem, welches oxidierte Derivat erwünscht ist, oder wenn sich ein gewünschtes Mischungsverhältnis eingestellt hat, wird die Inkubation unterbrochen. Dies kann beispielsweise durch Entfernen oder Abtöten der Mikroorganismen aus dem Kulturmedium erfolgen, beispielsweise durch Abzentrifugieren und Dekantieren und/oder durch Behandlung mit Säure, beispielsweise Trichloressigsäure, oder durch Behandlung mit Hitze.

Der oxidierte Aromat kann dann mit Hilfe üblicher Trennverfahren aus dem Kulturmedium, insbesondere der organischen Phase, isoliert werden, beispielsweise durch einfache Destillation, fraktionierte Destillation, Rektifikation, gegebenenfalls im Vakuum, oder durch Anwendung geeigneter chromatographischer Verfahren, bevorzugt aber durch Destillation.

f) Herstellung von 3,4-Dimethylbenzaldehyd:

Gemäß einer bevorzugten Ausführungsform der Erfindung wurden entsprechend obiger Kriterien für die organische Phase zur erfindungsgemäßen Oxidation aromatische Verbindungen und insbesondere zur Herstellung von 3,4-Dimethylbenzaldehyd aus Pseudocumol in einem zweiphasigen Fed-Batch-Prozess Bis-(2-ethylhexyl)phthalat (Dioctylphthalat) als organische Phase gewählt. Substrat und Produkt lösen sich sehr gut in Dioctylphthalat. Ausserdem ist diese billige organische Substanz schwer entflammbar und als zweite Phase für *E. coli* nicht toxisch. Der hohe Siedepunkt von Dioctylphthalat (380°C bei Normaldruck) erlaubt die Abtrennung der Produkte mittels einer Destillation.

Die Verteilungskoeffizienten von Pseudocumol, 3,4-Dimethylbenzylalkohol, 3,4-Dimethylbenzaldehyd und 3,4-Dimethylbenzoesäure zwischen Dioctylphthalat und M9 Medium als wässriger Phase sind in Tabelle 2 ersichtlich.

Tabelle 2: Verteilungskoeffizienten in DOP/M9 (Glucose) Zweiphasensystem

| Substanz | Verteilungskoeffizient | |
|---|---|---|
| Pseudocumol | 24300 | ± 1500 |
| 3,4-Dimethylbenzylalkohol | 50 | ±3 |
| 3,4-Dimethylbenzaldehyd | 906 | ± 20 |
| 3,4-Dimethylbenzoesäure | 0.188 | ± 0.005 |

Bei dem erfindungsgemäß bevorzugten zweiphasigen Fed-Batch-Prozess zur Herstellung von 3,4-Dimethylbenzaldehyd mittels rekombinanterXMO-exprimierenden *E. coli,* kommt der Kinetik dieses Mehrstufenprozesses eine entscheidende Bedeutung zu. So konnte beobachtet werden, dass die 3,4-Dimethylbenzoesäure (DBSäure) erst gebildet wird, wenn das Substrat nur noch in geringer Konzentration vorhanden ist (< 90 mM in der organischen Phase; entsprechend etwa 3.7 μM in der wässrigen Phase). Neben der höheren Affinität der Xylolmonooxygenase für Pseudocumol als für den entrsprechenden Aldehyd, trägt wahrscheinlich auch eine nicht kompetitive Inhibition des dritten Oxidationsschrittes durch Pseudocumol zu diesem Phänomen bei. Auch konnte bei Pseudocumolkonzentrationen über 150 mM in der organischen Phase (entsprechend etwa 6.2 μM in der wässrigen Phase) gleichzeitig eine 3,4-Dimethylbenzylalkohol- und eine 3,4-Dimethylbenzaldehydbildung beobachtet werden. Unter einer Pseudocumolkonzentration von 150 mM wird jedoch vor allem 3,4-Dimethylbenzaldehyd gebildet. Die 3,4-Dimethylbenzoesäurebildung wird ebenfalls inhibiert, wenn die Konzentration des Alkohols in der wässrigen Phase höher ist als diejenige des Aldehyd.

Bei fehlender XMO-Aktivität in rekombinanten *E. coli* wurde die Rückreaktion von 3,4-Dimethylbenzaldehyd zu 3,4-Dimethylbenzylalkohol beobachtet. Dies lässt sich auf unspezifische Alkoholdehydrogenaseaktivitäten in dem verwendeten *E. coli*-Stamm zurückführen. Wie im Fall der BADH liegt auch bei dieser Reaktion das Gleichgewicht auf der Seite des Alkohols (Bühler, Schmid et al. 2000).

Die Enzymaktivität hängt auch von der Glucosekonzentration im Medium ab. So haben sowohl hohe Glucosekonzentrationen im Medium als auch eine Glucoselimitation sowie eine Sauerstofflimitation eine inhibierende Wirkung auf die Enzymaktivität.

Gemäß einer bevorzugten Ausführungsform der Erfindung, welche in den folgenden Beispielen näher beschrieben wird, wurde 3,4-Dimethylbenzaldehyd aus Pseudocumol unter Verwendung von *E. coli*, in welchen die Gene der Xylolmonooxygenase von *Pseudomonas putida* mt-2 rekombinant eingesetzt wurden, in einer kinetisch kontrollierten Mehrschrittreaktion produziert. Dies wird durch die Fähigkeit der Xylolmonooxygenase ermöglicht, welche die Oxidation der Xylole über die entsprechenden Alkohole und Aldehyde in die korrespondierenden Säuren katalysieren kann. Die Kinetik dieser Mehrschrittreaktion konnte dazu genutzt werden, das Aldehyd spezifisch anzureichern. Unter Verwendung eines zweiphasigen Fed-Batch-Prozesses, in welchem das Edukt über eine organische Phase zugegeben wird, konnten im Pilotmassstab aus 30 Liter Ansatz 484 ml (96.5%) 3,4-Dimethylbenzaldehyd produziert und mittels Zentrifugation und Destillation aufgereinigt werden.

Darüber hinaus ist es erfindungsgemäß gelungen; das für die Umsetzung verwendete Kulturmedium weiter zu optimieren und prozessrelevante Parameter zu bestimmen und zu optimieren. Auch konnte aufgezeigt werden, dass das Plasmid und die XMO-Gene über 60 Stunden und 14 Generationen ohne Selektionsdruck stabil bleiben.

Ausgehend von obigen Ergebnissen für die Umsetzung von Pseudocumol, kann der Fachmann ohne unzumutbaren Aufwand die allgemeine Lehre der Erfindung auf die biokatalytische Umsetzung weitere Edukte der obigen Formel I ggf. unter Verwendung anderer Mikroorganismen übertragen. Er wird dabei insbesondere Abwandlungen in der Beschaffenheit des Reaktionsmediums (z.B. Puffer, Nährstoffe, organische Phase) und der Verfahrensbedingungen (z.B. Substratkonzentration, Fahrweise, Reaktortyp, Belüftung, Reaktionsdauer), Aufarbeitung des Reaktionsgemisches und dergleichen in Erwägung ziehen.

Die Erfindung wird nun anhand der folgenden nicht limitierenden Ausführungsbeispiele und unter Bezugnahme auf beiliegende Figuren näher erläutert. Dabei zeigt:

Figur 1: Plasmid pSZP3, reguliert durch das alk Regulatorsystem; alkBp, Promotor vom alk Operon; alkS, Gen für den positiven Regulator AlkS; xylM* und xylA, für die Xylolmonooxygenase kodierende Gene (* besagt, dass in xylM eine NdeI Schnittstelle entfernt wurde); Km Resistenzgen gegen Kanamycin; T4t, Transkriptionaler Terminatorvon Phage T4.

Figur 2: Zelltrockengewicht (CDW), Glucose- und Essigsäurekonzentration bei der Fermentation mit Feedlösung I mit Hefeextrakt

Figur 3: Konzentrationen von Pseudocumol, 3,4-Dimethylbenzylalkohol, 3,4Dimethylbenzaldehyd, 3,4-Dimethylbenzoesäure und Octan in derwässrigen und organischen Phase bei der Fermentation mit Feedlösung I mit Hefeextrakt

Figur 4: a) Zelltrockengewicht, Glucose- und Essigsäurekonzentration bei der Fermentation mit Feedlösung I ohne Hefeextrakt;
b) Konzentrationen von Pseudocumol, 3,4-Dimethylbenzylalkohol, 3,4Dimethylbenzaldehyd, 3,4-Dimethylbenzoesäure und Octan in derwässrigen und organischen Phase bei der Fermentation mit Feedlösung I ohne Hefeextrakt

Figur 5: Zelltrockengewicht, Glucose- und Essigsäurekonzentration bei der Fermentation mit erhöhter Substratkonzentration

Figur 6: Konzentrationen von Pseudocumol, 3,4-Dimethylbenzylalkohol, 3,4Dimethylbenzaldehyd, 3,4-Dimethylbenzoesäure und Octan in derwässrigen und organischen Phase bei der Fermentation mit erhöhter Substratkonzentration; Pfeil markiert gleichzeitige Reduktion der Feed- und der Belüftungsrate

Figur 7: Zelltrockengewicht (CDW), Glucose- und Essigsäurekonzentration bei der Aufnahme der Wachstumskurve von E. coli JM101 mit dem Riesenbergmedium

Figur 8: Zelltrockengewicht, Glucose- und Essigsäurekonzentration bei der Fermentation mit hoher Zelldichte bei Fed-Batchstart

Figur 9: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation mit hoher Zelldichte zu Beginn des Fed-Batchs; Pfeil markiert Zugabe von 1 % (v/v) Pseudocumol

Figur 10: Zelltrockengewicht, Glucose- und Essigsäurekonzentration bei der Fermentation mit normaler Zelldichte zu Beginn des Fed-Batchs

Figur 11: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation mit normaler Zelldichte zu Beginn des Fed-Batchs

Figur 12: Zelltrockengewicht, Glycerin- und Essigsäurekonzentration bei der Fermentation mit Glycerin als Kohlenstoff-Quelle

Figur 13: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation mit Glycerin als Kohlenstoff-Quelle.

Figur 14: Zelltrockengewicht, Glycerin- und Essigsäurekonzentrationen bei der Fermentation mit Glycerin als Kohlenstoff-Quelle und einer höheren Substratkonzentration

Figur 15: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation mit Glycerin als Kohlenstoff-Quelle und höherer Pseudocumolkonzentration

Figur 16: Zelltrockengewicht, Glucose- und Essigsäurekonzentration beim repetitiven Fed-Batch 1. Tag

Figur 17: Konzentrationen von Pseudocumol, 3,4-Dimethylbenzylalkohol, 3,4-Dimethylbenzaldehyd, 3,4-Dimethylbenzoesäure und Octan in der wässrigen und organischen Phase beim repetitiven Fed-Batch 1. Tag

Figur 18: Zelltrockengewicht, Glucose- und Essigsäurekonzentration beim repetitiven Fed-Batch 2. Tag

Figur 19: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase beim repetitiven Fed-Batch 2. Tag

Figur 20: Zelltrockengewicht, Glucose- und Essigsäurekonzentration bei der Fermentation im Pilotmassstab bei pH

6.6; Pfeil markiert Einstellung von pH 6.6

Figur 21: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation im Pilotmassstab

Figur 22: Zelltrockengewicht, Glucose- und Essigsäurekonzentration bei der Fermentation im Pilotmassstab bei pH 7.4

Figur 23: Konzentrationen von Pseudocumol, 3,4-Dimethylbenzylalkohol, 3,4-Dimethylbenzaldehyd, 3,4-Dimethylbenzoesäure und Octan in derwässrigen und organischen Phase bei der Fermentation im Pilotmassstab bei pH 7.4

Figur 24: Spezifische Aktivität und Aldehydbildungsrate bei der Fermentation im Pilotmassstab bei pH 7.4; Pfeil markiert Erhöhung des pH-Werts auf 7.4

Figur 25: Einflüsse prozessrelevanter Parameter auf den Bioprozess P: Pseudocumol, DBAlk: 3,4-Dimethylbenzylalkohol, DBAld: 3,4-Dimethylbenzaldehyd, DBSäure: 3,4-Dimethylbenzoesäure + →fördernder Einfluss; - →inhibierenden Einfluss

VERWENDETE ABKÜRZUNGEN:

| | |
|---|---|
| CDW | Zelltrockengewicht |
| DBAlk | 3,4-Dimethylbenzylalkohol |
| DBAld | 3,4-Dimethylbenzaldehyd |
| DBSäure | 3,4-Dimethylbenzoesäure |
| DOP | Dioctylphthalat |
| $NAD^+$ | β-Nicotinamid Adenindinucleotid, oxidierte Form |
| NADH | β-Nicotinamid Adenindinucleotid, reduzierte Form |
| P | Pseudocumol (Trimethylbenzol) |
| ppm | parts per million |
| rpm | Umdrehungen in einer Minute |
| v/v | Volumen pro Volumen |
| w/v | Gewicht pro Volumen |
| XMO | Xylenmonooxygenase |

MATERIAL UND METHODEN

1. Apparaturen

a) analytische Geräte

| | |
|---|---|
| GC Fisons Instruments | HRCG Mega 2 Series<br>Machery-Nagel, Önsingen, (CH)<br>Säule: Silikon Kapillarsäule OPTIMA-5 (25 m; innerer Durchmesser, 0,32mm, Filmdicke 0,25 μm) |
| GC | HP 5890 Serie 2 GC Plus<br>Säule: Kapillarsäule CARBOWAX 20M 25m * 0.25mm * 0.25mm (Macherey-Nagel) |
| Spektrophotometer | Novaspec II<br>Pharmacia LKB |

b) Geräte für die Biotransformation

| | |
|---|---|
| 3 Liter Rührreaktor | Case, RU Groningen, (NL) |
| 42 Liter Rührreaktor | New MBR, Zürich (CH) |
| | Regelung: pH, Temperatur, Rührgeschwindigkeit |
| | Betriebssystem: OS/9 |
| | Datenerfassung: Caroline II (PCS ,Wetzikon CH) |

c) Down-stream Geräte

| | |
|---|---|
| Zentrifuge | Cryofuge 8000 |
| | Heraeus Christ, Zürich (CH) |
| Vakuumpumpe | Typ D8B |
| | Leybold-Heraeus, Zürich (CH) |
| Kühlapparatur | 2219 Multitemp **II** Thermomatic Circulator |
| | LKB Broma Multitemp 11 |
| Vakuummessgerät | TypTR 216 |
| | Leybold Bakuum GmbH, Köln (De) |

2. Chemikalien

Die Chemikalien, die für die Experimente verwendet wurden, hatten analytische Qualität.

Für die Bestimmung der Glucose-, der Glycerin- und der Essigsäurekonzentration wurden folgende enzymatische Testkits verwendet und nach Angaben der Hersteller eingesetzt (Sigma Diagnostics, Boehringer Mannheim):

| | | |
|---|---|---|
| Glucosekit | Sigma Diagnostics | Glucose (Methode 315) |
| Glycerin UV-Test | Boehringer Mannheim | Glycerin (Art. Nr. 148270) |
| Essigsäure UV-Test | Boehringer Mannheim | Essigsäure ( Art. Nr. 148 261) |
| Glucose UV-Test | Boehringer Mannheim | Lactose/D-Glucose (Art. Nr. 986 119) |

3. Biologisches Material

a) Bakterien

Folgender Bakterienstamm wurde für die erfindungsgemäßen Experimente verwendet:
*E. coli* JM101 F, [traD26 proAB$^+$ *lacI$^q$, Δ(lacZ)M15] pro AB/supE, thi-1, Δ(lac proAB) (Sambrook, Fritsch et al. 1989)*

b) Plasmid

Folgendes Plasmid wurde für die erfindungsgemäßen Experimente verwendet (Figur 1): pSPZ3 alkS, AlkBp, xylMA, ori pMB1; Km$^r$ (Panke 1999)

4. Medien und Lösungen

a) Spurenelementlösung US* (Panke, 1999)

| | |
|---|---|
| In 1 Liter: | 1 M Salzsäure |
| | 1.5 g $MnCl_2$*4$H_2$0 |

(fortgesetzt)

| | |
|---|---|
| In 1 Liter: | 1 M Salzsäure |
| | 1.05 g $ZnSO_4$ |
| | 0.3 g $H_3BO_3$ |
| | 0.25 g $Na_2MoO_4*2H_2O$ |
| | 0.15 g $CuCl_2*2H_2O$ |
| | 0.84 g $Na_2EDTA*2H_2O$ |
| | 4.12 g $CaCl_2*H_2O$ |
| | 4.87 g $FeSO_4*7H_2O$ |
| (bei Verwendung des Riesenbergmediums 8.87 g $FeSO_4*7H_2O$) | |

b) LB-Medium (Luria Bertani Medium)

| | |
|---|---|
| In 1 Liter: | 10 g Trypton |
| | 5 g Hefeextrakt |
| | 5 g NaCl |

c) M9* Medium

| | |
|---|---|
| In 1 Liter: | 25.5 g $Na_2HPO_4*2H_2O$ |
| | 9 g $KH_2PO_4$ |
| | 1 g $NH_4Cl$ |
| | 1 g NaCl |

Der pH wurde mit NaOH auf 7.4 eingestellt

| | |
|---|---|
| komplettiert mit: | 0.5% (w/v) Glucose |
| | 2 ml $MgSO_4$ (1M) |
| | 1 ml Thiamin (1 % w/v) |
| | 1 ml Kanamycin (50mg/ml) |
| | 1 ml Spurenelementlösung US* |

d) M9 Medium:

| | |
|---|---|
| in 1 Liter: | 8.82 g $KH_2PO_4$ |
| | 10.85 g $K_2HPO_4$ |
| | 8.82 g $Na_2HPO_4$ |
| | 11.06 g $Na_2HPO_4*2H_2O$ |
| | 1 g $NH_4Cl$ |
| | 0.5 g NaCl |

Der pH wurde mit NaOH auf 7,1 eingestellt

| | |
|---|---|
| komplettiert mit: | 0.5% (v/v) Glucose |
| | 2 ml $MgSO_4$ (1M) |
| | 1 ml Thiamin (1% w/v) |
| | 1 ml Kanamycin (50 mg/ml) |
| | 1 ml Spurenelementlösung |

e) Riesenbergmedium:

| | |
|---|---|
| in 1 Liter | 13.3 g $KH_2PO_4$ |
| | 4.0 g $(NH_4)_2HPO_4$ |
| | 1.7 g Citronensäure |

Der pH wurde mit $NH_4OH$ auf 6,8 und mit NaOH auf 7,1 eingestellt

| | |
|---|---|
| komplettiert mit: | 0.7-2.5% (v/v) Glucose |
| | oder 0.7% (v/v) Glycerin |
| | 5 ml $MgSO_4$ (1M) |
| | 1 ml Kanamycin (50mg/ml) |
| | 1 ml Thiamin (1 % w/v) |
| | 5 ml-10ml Spurenelementlösung |

Die Medien, sowie die Stocklösungen von Magnesiumsulfat und Glucose wurden autoklaviert. Die Kanamycin- und Thiaminstocklösungen wurden sterilfiltriert.

f) Feedlösung I für M9 Medium:

| | |
|---|---|
| In 1 Liter | 45% (w/v) Glucose |
| | 9 g $MgSO_4$*7$H_2O$ |
| | (50 g Hefeextrakt) |

Der pH wurde mit HCl auf 3 eingestellt. Hefeextrakt wurde nicht in allen Versuchen verwendet.

g) Feedlösung II für Riesenbergmedium:

| | |
|---|---|
| In 1 Liter | 73% (w/v) Glucose |
| | 19.6g $MgSO_4$*7$H_2O$ |

Die Feedlösung wurde sterilfiltriert

h) organische Phase für die Umsetzung von Pseudocumol:

In 1 Liter Dioctylphthalat

(fortgesetzt)

20-40 ml Pseudocumol
10 ml Octan

i) Lösung 1

| | |
|---|---|
| In 100 ml | 333 µl 3 M NaAc pH 5,8 |
| | 2 ml 2.5 M $MnCl_2$ |
| | 100 µl 5 M NaCl |

k) Lösung 2

| | |
|---|---|
| In 50 ml | 167 µl 3 M NaAc pH 5,8 |
| | 8.3 ml 30% (w/v) Glycerin |
| | 3.5 ml 1 M $CaCl_2$ |
| | 200 µl 2,5 M $MnCl_2$ |

5. Molekularbiologische Methoden

5.1 Kompetente Zellen

Ein Schüttelkolben, welcher 50 ml LB Medium enthielt, wurde mit 500 µl Vorkultur angeimpft und über Nacht inkubiert. Nach erreichen einer $OD_{450}$ von 0,4 wurde die Kulturflüssigkeit in SS34 Tubes (40M) verteilt und 8 Minuten bei 8000 rpm bei 4 °C zentrifugiert. Der Überstand wurde verworfen. Die Zellen wurden in 8 ml obiger Lösung 1 resuspendiert und 20 Minuten auf Eis inkubiert. Danach wurden die Zellen durch eine 8-minütige Zentrifugation bei 8000 rpm von der Lösung 1 getrennt. Nachfolgend wurden sie auf Eis mit 800 µl obiger Lösung 2 resuspendiert und zu je 100 µl auf Eppendorf Proberöhrchen verteilt. Gelagert wurden sie bei -80°C.

5.2 Plasmidisolation

Für die Plasmidisolation wurde der E.Z.N.A.®Plasmid Miniprep Kit I (peQLab Biotechnologie GmbH) verwendet. 20 ml LB Medium, welches 1 % (w/v) Glucose und 20 µl Kanamycin (50mg/ml) enthielt wurde mit einer Kolonie transformierter *E. coli* JM101 beimpft und über Nacht bei 37°C inkubiert. Nach der Inkubation wurde die Kulturflüssigkeit auf 4 15 ml Tubes verteilt und zentrifugiert. Die nachfolgenden Arbeitsschritte wurde gemäss dem Protokoll des Miniprep Kit I durchgeführt. Mittels Gelelektrophorese wurde die Menge des erhaltenen Plasmids quantitativ abgeschätzt.

5.3 Transformation der Zellen

Die Transformation von *E. coli* JM101 mit dem Plasmid pSPZ3 wurde durch einen Hitzeschock erreicht. Es wurden 100 µl gefrorene kompetente Zellen 20 Minuten auf Eis aufgetaut. Nach Zugabe von 3 µl Plasmid wurden die Zellen auf Eis 10-20 Minuten inkubiert. Danach wurden sie während 3 Minuten bei 37°C einem Hitzeschock unterzogen und nach Ablauf der Zeit sofort wieder für 5 - 10 Minuten auf Eis gestellt. Nach der Zugabe von 1 ml LB Medium wurden die Zellen, bei 37°C für 1 Stunde inkubiert. Zuletzt wurden die Zellen auf LB Agarplatten, welche 20 µl Kanamycin (50 mg/ml) enthielten, ausplattiert und über Nacht bei 37°C inkubiert.

6. Biotransformationen im Labormassstab

Der zur Biotransformation verwendete Rührreaktor fasst ein Volumen von 3 Liter. Die Temperatur, der pH und die Rührgeschwindigkeit konnten automatisch geregelt werden. Der im Medium gelöste Sauerstoff (DOT) wurde mittels Sauerstoffelektrode gemessen und zeitabhängig aufgetragen.

6.1 Vorkulturen

1. Vorkultur: 4 ml LB Medium, welches 1 % (w/v) Glucose und 4 µl Kanamycin (50 mg/ml) enthielt, wurde mit einer Kolonie frisch transformierter *E. coli* JM101 (pSPZ3) angeimpft und über Nacht bei 30°C unter Schütteln inkubiert.

2. Vorkultur: Die 1. Vorkultur wurde in 100 ml 0,5% (w/v) Glucose oder Glycerin 200 µl Magnesiumsulfat (1 M), 100 µl Spurenelementlösung US*, 100 µl Thiamin (1 % w/v) und 100 µl Kanamycin (50 mg/ml) enthaltendes M9* Medium gegeben und während 10-12 Stunden bei 30°C unter Schütteln inkubiert.

6.2 Batch

900 ml Medium (M9 oder Riesenbergmedium), welches zusammen mit dem Bioreaktor sterilisiert und mit Thiamin, Spurenelementen, Glucose oder Glycerin, Magnesiumsulfat und wo angegeben mit Antibiotika komplettiert wurde, wurde mit 100 ml Vorkultur inokuliert. Die Rührgeschwindigkeit betrug 1500 rpm. Ausserdem wurde die Temperatur auf 30°C und der pH-Wert auf 7.1 durch Zugabe von 25 % $NH_4OH$ und 25% Phosphorsäure reguliert. Die Belüftungsrate betrug 1 l/min.

6.3 Fed-Batch

Nach Verbrauch der zugegebenen Glucose und der Verwertung der durch den Stoffwechsel der Bakterien produzierten Essigsäure (ca. 9-12 Stunden), wurde dem Medium eine Glucose oder Glycerin-Magnesiumsulfatlösung, welche zum Teil mit Hefeextrakt versetzt wurde, zugegeben. Die Glucosezufuhr variierte zwischen 4.5 g/h bis 11.25 g/h bei den verschiedenen Experimenten. Ebenfalls wurden 4 ml der Thiaminstocklösung und 4 ml der Spurenelementlösung US* zugegeben.

6.4 Biotransformation

Eine Stunde nach Starten des Fed-Batches wurde die organische Phase (enthielt 10 ml n-Octan als Induktor und 20 - 40 ml Pseudocumol) zugegeben. Gleichzeitig wurde die Rührgeschwindigkeit auf 2000 rpm erhöht. Die Luftzufuhr wurde bei dem Auftreten einer Sauerstofflimitation (DOT < 10%) der Bakterien auf 1,7 Liter/Minute erhöht. Bei Bedarf wurde die Rührgeschwindigkeit auf 2500 rpm erhöht. Die Biotransformation dauerte ca. 15-30 Stunden. Nach Beginn des Fed-Batchs wurden periodisch Proben entnommen und analysiert.

7. Biotransformation im Pilotmassstab

Zur Biotransformation im Pilotmassstab wurde der oben angegebene 42 Liter Rührreaktor eingesetzt. Rührgeschwindigkeit, Temperatur, pH-Wert und Überdruck im Reaktor wurden automatisch geregelt. Der Reaktor wurde automatisch vom OS/9 Betriebssystem mit dem Programm Caroline II (PCS, Wetzikon, Schweiz) gesteuert.

7.1 Vorkulturen

1. Vorkultur: 4 ml LB Medium, welches 1% (w/v) Glucose und 4 µl Kanamycin (50 mg/ml) enthielt, wurde mit einer Kolonie frisch transformierter *E. coli* JM101 (pSPZ3) angeimpft und über Nacht bei 30°C unter Schütteln inkubiert.

2. Vorkultur: Die 1. Vorkultur wurde in 100 ml und während 10-12 Stunden bei 30°C unter Schütteln inkubiert.

3. Vorkultur: Die 2. Vorkultur wurde in 2 l 0,5% (w/v) Glucose oder Glycerin 200 µl Magnesiumsulfat (1 M), 100 µl Spurenelementlösung US*, 100 µl Thiamin (1 % w/v) und 100 µl Kanamycin ( 50 mg/ml) enthaltendes M9* Medium gegeben.

7.2 Batch

Es wurden 14 Liter Riesenbergmedium im Reaktor sterilisiert. Nach der Sterilisation wurden das Medium mit Spurenelementen, Magnesiumsulfat, Thiamin und 0,7% (w/v) Glucose vervollständigt. Es wurde auf die Zugabe von

Kanamycin verzichtet. Der Rührreaktor wurde mit 1 -1,5 Liter Vorkultur beimpft. Die Rührgeschwindigkeit betrug 400 - 600 rpm. Belüftet wurde mit einer Rate von 20 l/min.

7.3 Fed-Batch

Nach Verbrauch der zugegebenen Glucose und der von den Bakterien gebildeten Essigsäure, wurde die Feedlösung II zugeführt. Die Feedrate betrug zu Beginn 180 g/h wurde jedoch bei einer Glucoselimitation erhöht. Ausserdem wurde 15 ml n-Octan zur Induktion der Zellen, 60 ml Thiamin (1 % w/v) und 60 ml Spurenelementlösung US* zugegeben.

7.4 Biotransformation

Nach 1 Stunde wurden 15 Liter Dioctylphthalat als organische Phase, die 4,3% (v/v) Pseudocumol und 1 % (v/v) n-Octan enthielt, zugegeben. Die Rührgeschwindigkeit wurde auf 1000 rpm erhöht. Ebenso wurde die Belüftung auf 40 l/min erhöht. Es wurden periodisch Proben gezogen und auf deren Gehalt an Substrat, Produkt, Nebenprodukt und Octan analysiert. Ebenso wurde das Zelltrockengewicht, die Glucosekonzentration und die Essigsäurekonzentration bestimmt. In Abbildung 7 ist der Ablauf des Prozesses schematisch dargestellt.

8. Bestimmung der Verteilungskoeffizienten im Zweiphasensystem

Der Verteilungskoeffizient ist definiert als die Konzentration der Substanz in der organischen Phase im Verhältnis zur Konzentration der Substanz in der wässrigen Phase. Zur Bestimmung der Verteilungskoeffizienten vom Riesenbergmedium mit Glycerin und Dioctylphthalat wurden verschiedene Konzentrationen von Pseudocumol, 3,4-Dimethylbenzylalkohol, 3,4-Dimethylbenzaldehyd und 3,4-Dimethylbenzoesäure zu 1 : 1- Mischungen aus Dioctylphthalat und Riesenbergmedium gegeben. Durch starkes Schütteln und Inkubation über Nacht wurde das Verteilungsgleichgewicht eingestellt.

Durch Zentrifugation wurden wässrige und organische Phasen separiert. Mit Etherwurden die organische Phasen verdünnt und die wässrige Phase extrahiert und mittels GC analysiert. Anhand der Messungen wurden die Konzentrationen in den 2 Phasen bestimmt und die Verteilungskoeffizienten berechnet. Es wurde je eine Dreifachbestimmung durchgeführt.

9. Probenanalysen

9.1 GC-Analyse

Die wässrige und die organische Phase wurden durch zentrifugieren bei 20°C separiert. Danach wurde eisgekühlter Ether mit 0.1 mM Dodecan als interner Standard den Proben zugegeben. Nach der Zugabe einer sättigenden Konzentration von Natriumchlorid wurde die wässrige Phase durch starkes Schütteln 1 Minute extrahiert und anschliessend die Phasen durch zentrifugieren separiert. Die organische Phase werden durch Zugabe von Natriumsulfat getrocknet und analysiert. Mittels GC (Nagel, Oensingen, Schweiz) wurde der Gehalt an Substrat, Produkt, Nebenprodukte und Octan bestimmt. Die Probeneinspritzung geschah ohne Splitt. Als Trägergas wurde Wasserstoff verwendet. Für die Messung wurde folgendes Temperaturprogramm eingesetzt: 2 min bei 40°C; von 40 auf 70°C in einer Aufheizrate von 15°C/min; von 70 auf 105°C bei einer Aufheizrate von 5°C/min und von 105°C auf 280°C bei einer Aufheizrate von 20°C/min; 5 min bei 280°C. Die chemischen Verbindungen wurden mit einem Flammenionisationsdetektor gemessen. Die Werte von Substrat, Produkt und Nebenprodukten wurden in einer Grafik dargestellt. Ebenso wurden die spezifische Aktiviät und die Aldehydbildungsrate graphisch dargestellt. Dazu wurden folgende Berechnungen durchgeführt:

$$\text{spezifische Aktivität} = \text{Unit} / \text{g Zelltrockengewicht}$$

Ein Unit ist die Aktivität die 1 μmol Gesamtprodukt (DBAlk, DBAld, DBSäure) in 1 Minute produziert.

$$\text{Aldehydbildungsrate} = 1\ \mu\text{mol gebildetes Aldehyd in 1 Minute} / \text{g Zelltrockengewicht}$$

9.2 Bestimmung der optischen Dichte

Die Zellen wurden mittels Zentrifugation bei 4°C abgetrennt. Das Volumen derwässrigen Phase wurde auf dem Proberöhrchen eingezeichnet und anschliessend wurde der Überstand abgenommen und verworfen. Das Zellpellet wurde in einem, zu der wässrigen Phase äquivalentem, Mediumvolumen aufgenommen und resuspendiert. Die optische Dichte wurde mittels eines Spectrophotometers bei 450 nm gemessen.

9.3 Glucose-, Glycerin-, und Essigsäurebestimmung

a) Glucose-, Glycerin, und Essigsäurekonzentrationen in derwässrigen Phase wurde mit Hilfe obiger enzymatischer Test-Kits bestimmt.

b) Essigsäurebestimmung mittels GC
50 µl wässriger Phase wurden 350 µl Wasser, 50 µl $H_3PO_4$ (85%) und 50 µl 100 mM Natriumbutyrat als interner Standart, zugegeben. Dieses wurde mittels eines HP 5890 Series 2 GC analysiert. Das verwendete Temperaturprogramm sah wie folgt aus: 0.5 min bei 85°C, von 85 - 100°C bei einer Aufheizrate von 12°C/min, von 110 - 200°C in einer Aufheizrate von 79°C/min, 2.5 min bei 200°C. Als Trägergas wurde Helium verwendet.

9.4 Bestimmung der Plasmidstabilität

Die Proben wurden zentrifugiert und die Zwischenphase von der wässrigen und der organischen Phase auf dem Proberöhrchen gekennzeichnet. Der Überstand wurde verworfen und das Zellpellet mit einem äquivalentem Volumen der wässrigen Phase mit Medium resuspendiert. Die Probe wurde verdünnt und auf LB-Agarplatten, LB-Agarplatten mit 20 µl Kanamycin (50 mg/ml) und LB-Agarplatten mit 20 µl Kanamycin (50 mg/ml) und 20 µl Indol auspfattiert. Exprimieren die Zellen die XMO-Gene, so verfärben sich die Kolonien auf den LB-Indolagarplatten blau. Dies lässt sich durch die Tatsache begründen, da die XMO-Gene die Oxidation von Indol zu Indigo katalysieren. Die Platten wurden bei 30°C über Nacht inkubiert und anschliessend ausgezählt. Es wurden die Lebendkeimzahlen miteinander verglichen.

10. Aufarbeitungsprozess (Down-Stream Prozess)

10.1 Zentrifugation

Nach dem Abernten des Reaktors wurden die organische und die wässrige Phase mittels einer Heraeus Zentrifuge 15 Minuten bei 5000 rpm und 4°C separiert. Die wässrige Phase wurde verworfen, während die organische Phase mit Natriumsulfat (1 kg/15 l organische Phase) getrocknet und bei 5°C gelagert wurde. Das Natriumsulfat wurde vor der Destillation abfiltriert (mit Papierfilter).

10.2 Destillation

Die organische Phase im Kolben wurde auf 200°C aufgeheizt bei einem Feinvakuum zwischen 0.08 mbar und 0.15 mbar. Das Destillat wurde in drei Fraktionen aufgeteilt; in einen Vorlauf (110°C - 125°C), eine Hauptlauf (120°C - 170°C) und einen Nachlauf (170°C - 200°C). Das Vakuum und die Temperatur wurden automatisch geregelt.

I. VERSUCHE ZUR BIOTRANSFORMATION VON PSEUDOCUMOL IM LABORMASSSTAB

Die Biotransformationen im Labormassstab (Beispiele 1, 2 und 3) dienten einerseits dazu, die prozessrelevanten Parameter zu bestimmen und zu optimieren und andererseits zur Mediumoptimierung. Als Ziel galt die Steigerung der Produktions-Menge von 3,4-Dimethylbenzaldehyd (DBAld) während der Biotransformation.

Des weiteren wurde bei den Biotransformationen die Bildung von Essigsäure, welche von den Zellen als Stoffwechselprodukt produziert und ins Medium abgegeben wird, untersucht. Dieses inhibiert bei einer zu hohen Konzentration das Biomassenwachstum. Durch Einsatz eines neuen Mediums (Riesenbergmedium) wurde auf das Erreichen von höheren Zelldichten und somit auf eine höhere volumetrische Enzymaktivität abgezielt. Zur Optimierung des Mediums kann auch die Variation der Feedlösung gezählt werden. Es wurden Biotransformationen unternommen, in denen der Feedlösung kein Hefeextrakt zugegeben wurde, da dieser unter anderem undefinierte Inhaltsstoffe enthält, welche die Reproduzierbarkeit der Biotransformation erschweren. Ebenso wurde der Einfluss der beiden Kohlenstoff-Quellen Glucose und Glycerin auf die Enzymaktivität und Essigsäurebildung gestestet.

Außerdem wurde die Stabilität des Plasmids und der XMO-Gene ohne Selektionsdruck untersucht. So wurden Biotransformationen ohne Kanamycin durchgeführt.

Zur Produktion von DBAld wurde ein zweiphasiger Fed-Batch-Prozess getestet. Dabei wurde nach einem Batch der Fed-Batch gestartet und danach die organische Phase, welche das Substrat und den Induktor enthielt, zugegeben wurde. Die Figuren zeigen die erhaltenen Resultate von Beginn des Fed-Batchs an. In Graphiken in welchen die Konzentrationen von Substrat, Produkt, Nebenprodukt und Octan abgebildet sind, wurden die Konzentrationen der Substanzen in der wässrigen und organischen Phase addiert. Die spezifische Aktivität ist als 1 μmol gebildetes Gesamtprodukt (DBAlk, DBAld und DBSäure) in einer Minute pro g Zelltrockengewicht definiert. Die Aldehydbildungsrate entspricht 1 μmol gebildetes 3,4-Dimethylbenzaldehyd in einer Minute pro g Zelltrockengewicht.

Beispiel 1: Biotransformationen mit M9 Medium

Experiment 1.1: Fermentation mit Feedlösung I mit Hefeextrakt

Die Biotransformation wurde nach dem beschriebenen Fermentationsprozess durchgeführt. Dem Medium wurde vor der Inokulation 0,5% (w/v) Glucose zugegeben. Nach dem Batch erreichte die Biomasse ein Zelltrockengewicht von 3.1 g/l. Die Feedrate betrug 10 g/h beim Start des Fed-Batches, was einer Glucosezufuhr von 4,5 g/h entspricht. Um eine zu starke Glucoselimitation zu verhindern, wurde die Feedrate stufenweise erhöht. Die Belüftungsrate wurde nach 3 Stunden von 1 l/min auf 1,7 l/ min erhöht um eine Sauerstofflimitation zu umgehen.

In Figur 2 sind die Werte der Essigsäure- und Glucosekonzentration und des Zelltrockengewichts ersichtlich. Das Zelltrockengewicht betrug nach Erreichen der stationären Phase 19 g/l. Zur Zeit des Wachstumsstops der Bakterien, nach 8 Stunden betrug die Essigsäurekonzentration 10 g/l während die Glucosekonzentration 9 g/h betrug.

In Figur 3 sind die Änderungen der Konzentrationen von Pseudocumol, 3,4-Dimethylbenzaldehyd (DBAlk), 3,4 Dimethylbenzylalkohol (DBAlk), 3,4-Dimethylbenzoe-säure (DBSäure) und Octan dargestellt. Die Konzentration von Pseudocumol betrug bei Zugabe der organischen Phase 1.6% (v/v). Nach einer Induktionsphase von 1.5 Stunden wurde vor allem 3,4-Dimethylbenzaldehyd gebildet. Bei Erreichen einer Substratkonzentration von 90 mM begann sich DBSäure zu bilden. Nach 10 Stunden wurde eine Säurekonzentration von 22 mM, welche für den Rest der Biotransformation konstant blieb, erreicht. Erst nach 10 Stunden wurde DBAlk gebildet. Am Ende der Biotransformation nach 30 Stunden betrug die Konzentration von DBAld 43 mM und diejenige von DBAlk 20 mM.

Die spezifische Aktivität sowie die Aldehydbildungsrate erreichten nach 4 Stunden die maximalen Werte von 12.6 U/g CDW beziehungsweise 9.8 U/g CDW. Die spezifische Aktivität nahm danach kontinuierlich ab. Die Aldehydbildungsrate wies jedoch 5.5 Stunden nach Starten des Fed-Batches eine starke Abnahme auf, dies entsprach dem Zeitpunkt an dem die Bildung von 3,4-Dimethylbenzoesäure einsetzte. Nach 8 Stunden nahm die Aldehydbildungsrate wieder zu.

Anhand der erhaltenen Resultate kann bestätigt werden, dass die 3,4-Dimethylbenzoesäurebildung bei einer Pseudocumolkonzentration von 90 mM einsetzt.

Experiment 1.2: Fermentation mit Feedlösung I ohne Hefeextrakt

Ziel dieser Fermentation war es nun die Auswirkungen der Feedlösung I ohne Hefeextrakt auf die Biotransformation zu untersuchen.

In Figur 4a sind die Verläufe der Glucose- und Essigsäurekonzentration und des Zelltrockengewichts dargestellt. Die Glucosekonzentration im Medium betrug vorder Inokulation 0.5% (w/v). Zu Beginn des Fed-Batches wies das Zelltrockengewicht eine Konzentration von 3 g/l auf. Die Feedlösung ohne Hefeextrakt wurde in einer konstanten Rate von 10 g/h zugeführt. Dies entspricht einer Glucosezufuhrrate von 4.5 g/h. Nach 7 Stunden wurde die Rührgeschwindigkeit von 2000 rpm auf 2500 rpm erhöht um eine Sauerstofflimitation zu umgehen (DOT >10%).

Schon geringe Glucosekonzentrationen hatten eine Essigsäurebildung zu folge. So konnte die Konzentration von Essigsäure bei Glucoselimitation konstant auf einen Wert von 1 g/l gehalten werden. Bei Erhöhung der Glucosekonzentration im Medium nahm die Essigsäure kontinuierlich bis zu einer Konzentration von 26.6 g/l zu. Am Ende der Biotransformation betrug die Biomassekonzentration 12 g/l.

Die Substratkonzentration in der organischen Phase betrug 1.6% (v/v) (Figur 4b). 2 Stunden nach Zugabe der organischen Phase begann sich 3,4-Dimethylbenzaldehyd zu bilden. Ab einer Substratkonzentration von 90 mM wurde jedoch hauptsächlich DBSäure gebildet, während die 3,4-Dimethylbenzaldehydkonzentration konstant blieb. Erst nach 10 Stunden setzte die Bildung von DBAld wieder ein und erreichte eine Konzentration von 41 mM. Bei niedrigerer Enzymaktivität im zweiten Teil der Biotransformation (ab 15 Stunden) wurde DBAlk gebildet.

Die spezifische Aktivität erreichte 3 Stunden nach Start des Fed-Batch den Höchstwert von 10 U/g CDW, brach danach jedoch ein. Nach einer geringen Zunahme nahm die spezifische Aktivität 9 Stunden nach Beginn des Fed-Batchs kontinuierlich ab. Auch die Aldehydbildungsrate erreicht nach 3 Stunden das Maximum von 7.1 U/g CDW. Wie im vorher

beschriebenen Experiment wurde zu Beginn der 3,4-Dimethylbenzoesäurebildung ein Einbruch der Aldehydbildungsrate beobachtetet.

Diese Fermentation zeigt, dass das Weglassen des Hefeextrakts auf das Biomassenwachstum und auf die Enzymaktivität einen grossen Einfluss hat. So wurden eine geringere Zelltrockenmasse wehrend des Fed-Batches erreicht als im vorhergehenden Vorversuch. Ebenso wurde eine niedrigere spezifische Aktivität und Aldehydbildungsrate beobachtet. Im Vergleich mit dem vorhergegangenen Versuch wurde eine langsamere Abnahme der spezifischen Aktivität festgestellt. Die Essigsäurekonzentrationen im Medium waren ebenso niedriger als im vorhergegangenen Versuch.

Experiment 1.3: Fermentation mit erhöhter Substratkonzentration

In früheren Versuchen konnte festgestellt werden, dass eine Erhöhung der Substratkonzentration auf die Bildung von 3,4-Dimethylbenzaldehyd einen positiven Einfluss aufweist. So wurde die Substratkonzentration von 1.6% (v/v) auf 3.1 % (v/v) erhöht. Des weiteren wurde versucht die Glucosekonzentration und somit auch die Essigsäurekonzentration im Medium tief zu halten. Im 2. Teil der Biotransformation, 15 Stunden nach Start des Fed-Batchs wurde der Effekt einer gleichzeitig auftretenden starken Glucose-und Sauerstofflimitation untersucht. So wurde die Feedzufuhr und die Belüftung gleichzeitig reduziert.

Vor der Inokulation wurde dem Medium 0.5% (w/v) Glucose zugegeben. Ausgehend von einem Zelltrockengewicht von 3 g/l nahm die Biomasse während des Fed-Batchs bis auf eine Konzentration von 27 g/l zu. Zu Beginn des Fed-Batchs wurde 10 g/h Feedlösung mit Hefeextrakt zugeführt (Glucosezufuhrrate 4.5 g/h). Die Feedrate wurde im Folgenden stufenweise erhöht. Die Glucoselimitation wurde jedoch erhalten, um hohe Essigsäurekonzentration im Medium zu verhindern. Die Konzentration von Essigsäure im Medium konnte über längere Zeit tief gehalten werden. Dies hatte sogar eine Verwertung der Essigsäure zur Folge (Figur 5). Um eine Sauerstofflimitation zu verhindern wurden die Belüftungsrate von 1 l/min auf 1.5 l/min und die Rührerdrehzahl von 2000 rpm auf 2500 rpm erhöht. Nach 16 Stunden wurde gleichzeitig die Feedrate und die Belüftungsrate reduziert, was eine starke Glucose- und Sauerstofflimitation und einen Anstieg der Essigsäurekonzentration zur Folge hatte.

Die Biotransformation wurde mit einer Substratkonzentration von 3.1% (v/v) gestartet. 1 Stunde nach der Induktion der Zellen setzte die Bildung von 3,4-Dimethylbenzaldehyd (BDAld) und 3,4-Dimethylbenzylalkohol (DBAlk) ein. 16 Stunden nach Start des Fed-Batchs, nach der Reduzierung der Feedrate und der Belüftungsrate, konnte eine Rückbildung des DBAld in DBAlk durch unspezifischen Alkoholdehydrogenasen von *E. coli* festgestellt werden (Figur 6). Die Konzentration von DBAlk betrug nach 30 Stunden 130 mM während die DBAld-Konzentration lediglich bei 30 mM lag. Während der Biotransformation konnte eine geringe 3,4-Dimethylbenzoesäurebildung festgestellt werden.

Nach Erreichen des Höchstwertes der spezifischen Aktivität von 18.6 U/g CDW 8 Stunden nach Start des Fed-Batchs nahm die spezifische Aktivität ab. Dies ist einerseits auf den Wachstumsstop der Zellen zu diesem Zeitpunkt, andererseits auf die Abnahme der Substratkonzentration zurückzuführen. Die Aldehydbildungsrate erreichte ein Maximum von 16 U/g CDW. Im Gegensatz zu der Fermentation mit Feedlösung I mit Hefeextrakt und der Fermentation, in welcher der Feedlösung I kein Hefeextrakt beigegeben wurde, war kein Einbruch der Aktivität nach 5 Stunden beobachtbar.

Die durchgeführte Fermentation hat gezeigt, dass höhere Substratkonzentrationen eine höhere Enzymaktivität zur Folge haben. Da die Essigsäurebildung während der Biotransformation tief gehalten werden konnte, wurde ein höhere Zelldichte während des Fed-Batchs erreicht. Anhand der erhaltenen Resultate kann gesagt werden, dass eine gleichzeitig auftretende Glucose- und Sauerstofflimitation einen Verlust der Xylolmonooxygenaseaktivität zur Folge hat. Die unspezifischen Alkoholdehydrogenasen von *E. coli* bildeten 3,4-Dimethylbenzaldehyd in 3,4-Dimethylbenzylalkohol zurück.

Beispiel 2: Biotransformation mit dem Riesenbergmedium

Um eine höhere Zelldichte und somit eine höhere volumetrische Aktivität zu erhalten, wurde ein neues, in der Literatur als Hochzelldichtemedium bezeichnetes Medium verwendet (Riesenberg 1991). Dieses Medium weist gegenüber dem bis dahin verwendeten M9 Medium weniger Phosphatsalze auf. Ausserdem enthält das Medium mehr Stickstoff, da unter anderem zum Einstellen des pH-Wertes Ammoniakwasser verwendet wird. Die Feedlösung II, welche zusammen mit dem Riesenbergmedium verwendet wurde, enthält im Gegensatz zur Feedlösung I keinen Hefeextrakt.

Experiment 2.1: Wachstumskurve von E. coli JM101 (PSZP3) mit Riesenbergmedium

Um das Wachstumsverhalten von *E. coli* JM101(pSZP3) und die Bildung des Stoffwechselprodukts Essigsäure bei der Verwendung des Riesenbergmediums zu untersuchen, wurde zuerst eine Wachstumskurve aufgenommen. Die Stabilität des Plasmids und der XMO-Gene wurden Anhand von Indolagarplatten und Kanamycinagarplatten überprüft. So konnte getestet werden, ob das Plasmid über Generationen weitergegeben und exprimiert wird.

Das Batchmedium enthielt 2.5% (w/v) Glucose. Der Batch dauerte mit dem Abbau der gebildeten Essigsäure 15 Stunden (Figur 7). Das Zelltrockengewicht betrug nach der Inokulation 0.18 g/l und nahm bei einer Wachstumsrate von 0.4 $h^{-1}$ bis auf 15 g/l zu. Während des Batchs wurde 1 g/l Essigsäure gebildet.

Das Wachstumsverhalten der Zellen unter Verwendung des Riesenbergmediums war ähnlich wie dasjenige mit M9 Medium. Es konnte eine geringe Essigsäurebildung während des Batchs festgestellt werden. Auch die Zeitspanne in welcher die Essigsäure von den Bakterien wieder verwertet wurde war vergleichsweise kurz. Anhand der Koloniezahlen auf den LB-Agarplatten, den LB-Agarplatten mit Kanamycin und den LB-Agarplatten mit Indol und Kanamycin kann gesagt werden, dass das Plasmid und die XMO Gene stabil blieben. So konnte kein signifikanter Unterschied der gewachsenen Kolonien auf den verschiedenen LB-Agarplatten festgestellt werden. Ebenso fiel die Farbreaktion auf den LB-Agarplatten mit Indol positiv aus.

Experiment 2.2: Fermentation mit hoher Zelldichte zu Beginn des Fed-Batchs

Nach der Aufnahme der Wachstumskurve wurde das Riesenbergmedium zur Biotransformation eingesetzt. Besonderes Augenmerk wurde dabei auf das Biomassenwachstum und in diesem Zusammenhang auf die Essigsäurebildung gerichtet.

Ziel der Fermentation war es, durch eine hohe Anfangszelldichte bei Start des Fed-Batchs eine höhere Biomassenkonzentration am Ende der Biotransformation zu erreichen. Dies hätte eine Steigerung der volumetrischen Aktivität während der Biotransformation zur Folge.

Um eine vergleichsweise hohe Zelldichte nach dem Batch zu erreichen, wurde dem Medium anstelle von 0.5% (w/v) 2.5% (w/v) Glucose zugegeben. Zu Beginn des Fed-Batchs betrug das Zelltrockengewicht 16 g/l (Figur 8). Die Biomasse nahm während des Fed-Batchs nicht mehr stark zu und erreichte eine Höchstkonzentration von 24.7 g/l.

Dem Bioreaktor wurde während des Fed-Batchs konstant 12.5 g/h Feedlösung II zugeführt. Dies entspricht einer Glucosezufuhrrate von 9 g/h. Da die Zellen bereits nach 2 Stunden sauerstofflimitiert waren und die Rührgeschwindigkeit sowie die Belüftungsrate bereits auf das Maximum eingestellt waren, wurde anstelle von Luft mit Sauerstoff belüftet. Mit dieser Massnahme konnte der im Medium gelöste Sauerstoff (DOT) über einen Wert von 10% gehalten werden. Die Zellen waren ab der 5. Stunde nicht mehr glucoselimitiert. Die Glucosekonzentration blieb zwischen der 5. Stunde nach Start des Fed-Batchs und der 15. Stunde konstant bei 2 g/l, nahm danach jedoch bis auf 25 g/l zu. Da die Glucosekonzentration im Medium vergleichsweise hoch war, stieg die Essigsäurekonzentration im Medium kontinuierlich an und erreichte am Ende des Fed-Batchs einen Wert von 30 g/l.

Mit Beginn des Fed-Batchs wurde dem Medium 1 Liter organische Phase mit einer Substratkonzentration von 3.4% (v/v) zugegeben.

Aus Figur 9 ist ersichtlich, dass die Bildung von 3,4-Dimethylbenzylalkohol sofort einsetzte. Die 3,4-Dimethylbenzaldehydbildung setzte nach 2 Stunden ein. 11 Stunden nach Start des Fed-Batchs wurde nochmals 1 % (v/v) Pseudocumol zugegeben. Dieses konnte jedoch nur noch zum Teil umgesetzt werden. Die Endkonzentration von 3,4-Dimethylbenzaldehyd betrug 113 mM. Pseudocumol und 3,4-Dimethylbenzylalkohol wiesen bei Abbruch der Biotransformation eine Konzentration von 65 mM auf. Die 3,4-Dimethylbenzoesäure wurde kaum gebildet.

3 Stunden nach Start des Fed-Batchs waren die Zellen voll induziert. Die spezifische Aktivität (Höchstwert 13 U/g CDW) und die Aldehydbildungsrate (Höchstwert 10 U/g CDW) waren niedrig. Die spezifische Aktivität nahm 4 Stunden nach Wachstumsende der Zellen stark ab. Nach der Substratzugabe wurde ein Anstieg auf 6 U/g CDW beobachtet. Auch die Aldehydbildungsrate nahm 4 Stunden nach Wachstumsende stark ab und blieb tief.

Der Start des Fed-Batchs mit höherer Anfangszelldichte führte unter den gewählten Bedingungen nicht zu höherer Zelldichte während des Fed-Batchs. Die Biomasse nahm lediglich während 3 Stunden zu. Es konnte auch ein Verlust der Enzymaktivität beobachtet werden. Während dem Fed-Batch wurde viel Essigsäure gebildet.

Experiment 2.3: Fermentation mit normaler Zelldichte zu Beginn des Fed-Batchs

In der vorhergehenden Fermentation konnte trotz hoher Zelldichten zu Beginn des Fed-Batchs keine höhere Zelldichte erreicht werden. Die hohe Anfangszelldichte hatte auf die Biotransformation eher negative Einflüsse. So war die spezifische Aktivität niedrig. Auch konnte eine kurze Aldehydbildungsdauer verzeichnet werden. Aus diesem Grund wurde der Fed-Batch mit geringerer Zelldichte gestartet.

Es wurde anstelle von 2.5% (w/v) Glucose nur 0.7% (w/v) Glucose zugegeben. Ausgehend von einer Biomassenkonzentration von 4.1 g/l erreichte das Zelltrockengewicht am Ende des Fed-Batchs 22 g/l. Die Glucose wurde mit einer konstanten Rate von 9 g/h dem Bioreaktor zugeführt. Die Essigsäurekonzentration betrug ca. 7 g/l nach 9 Stunden. Zu diesem Zeitpunkt befanden sich die Bakterien in der stationären Phase (Figur 10).

Gleichzeitig mit Beginn des Fed-Batchs wurde dem Medium 0.1% (v/v) Octan zugegeben. 1 Stunde später wurde die organische Phase mit 4% (v/v) Pseudocumol und 1 % (v/v) n-Octan zugegeben. 2 Stunden nach Beginn des Fed-Batchs waren die Zellen voll induziert und 3,4-Dimethylbenzylalkohol- und 3,4-Dimethylbenzaldehyd begann sich

in der organischen Phase zu akkumulieren. Während sich anfangs der Alkohol schneller anreicherte als das Aldehyd wurde ab einer Substratkonzentration von 150 mM 3,4-Dimethylbenzaldehyd gebildet. 3,4-Dimethylbenzylalkohol wurde bei tiefer Pseudocumol-Konzentrationen in 3,4-Dimethylbenzaldehyd umgewandelt. Die Endkonzentration von 3,4-Dimethylbenzaldehyd betrug am Ende der Biotransformation 200 mM. Bei tiefer Pseudocumol- und 3,4-Dimethylbenzylalkohol-Konzentration setzte die 3,4-Dimethylbenzoesäurebildung ein (Figur 11).

Die spezifische Aktivität betrug 3 Stunden nach dem Start des Fed-Batchs 26 U/g CDW. Danach nahm sie parallel zur Substratkonzentration ab. Nach 5 Stunden erreichte die Aldehydbildungsrate den Höchstwert von 14 U/g CDW. Sie nahm danach kontinuierlich ab.

Die Substratkonzentration und die Anfangszelldichte wurden optimal gewählt um einen Komplettumsatz zu 3,4-Dimethylbenzaldehyd zu erreichen. Bei tieferer Anfangszelldichte konnte länger eine Aldehydbildungsrate erreicht werden. Das Riesenbergmedium, welches keinen Hefeextrakt in der Feedlösung enthält, erlaubt das Erreichen derselben spezifischen Aktivität und Aldehydbildungsrate wie das bei Verwendung des M9 Mediums nur mit Hefeextrakt in der Feedlösung möglich ist.

Experiment 2.4: Fermentation mit Glycerin als C-Quelle

Der Einsatz von Glycerin als C-Quelle birgt möglicherweise einige Vorteile gegenüber Glucose als C-Quelle. So kann eine geringere Essigsäurebildung und eine höhere Enzymaktivität erwartet werden. Deshalb wurde der Einfluss von Glycerin als C-Quelle auf die Biotransformation untersucht.

Vor der Inokulation wurde dem Medium 0.7% (v/v) Glycerin zugegeben. Die Biomasse wies am Ende des Batchs eine Konzentration von 4.2 g/l auf. Es konnte wie erwartet ein langsameres Biomassenwachstum als bei Verwendung von Glucose als C-Quelle beobachtet werden. Nach Erreichen der stationären Phase betrug das Zelltrockengewicht 19 g/l. Gleichzeitig mit dem Beginn des Fed-Batchs wurde dem Medium 0.1% (v/v) Octan zugegeben. Nach einer anfänglichen Feedrate von 7.7 g/h (Glycerinzufuhrrate von 7.2 g/h) wurde diese stufenweise erhöht um eine Glycerinlimitation zu verhindern. Die Essigsäure begann sich erst in der stationären Phase nach 9.5 Stunden zu bilden und erreichte am Ende des Fed-Batchs einen Wert von 18 g/l (Figur 12).

Die Substratkonzentration betrug bei der Zugabe der organischen Phase 4% (v/v). 3,4-Dimethylbenzylalkohol wurde von Beginn weg gebildet. Ab einer Pseudocumolkonzentration von 240 mM wurde ausschliesslich 3,4-Dimethylbenzaldehyd gebildet. Der 3,4-Dimethylbenzylalkohol wurde direkt in Aldehyd umgeformt. Die Säurebildung setzte bei einer Substratkonzentration um 150 mM ein. Wie aus Figur 13 ersichtlich ist, erreichte das 3,4-Dimethylbenzaldehyd eine Endkonzentration von 144 mM.

Die maximale Bildungsrate von 3,4-Dimethylbenzaldehyd war sehr hoch (32.7 U/g CDW). Die spezifische Gesamtaktivität erreichte einen Wert von 33 U/g CDW. Die hohen Aktivitäten wurden jedoch nicht lange gehalten. Schon nach 11 Stunden betrug die spezifische Aldehydbildungsrate nur noch 2.5 U/g CDW.

Aus diesen Resultaten folgt, dass bei Einsatz von Glycerin als C-Quelle höhere Enzymaktivitäten ab 30 U/g CDW erreicht wurden. Jedoch konnte die Aktivität nur während des Biomassenwachstums aufrecht erhalten werden.

Neben der höheren Enzymaktivität konnte eine andere Kinetik im Gegensatz zur Verwendung von Glucose als C-Quelle beobachtet werden. So hat sich die alleinige 3,4-Dimethylbenzaldehydakkumulation im Medium schon bei einer Dimethylbenzaldehydakkumulation im Medium schon bei einer Pseudocumolkonzentration von 240 mM eingestellt, im Gegensatz zu 150 mM bei Verwendung von Glucose als C-Quelle. Auch die 3,4-Dimethylbenzoesäurebildung setzt bereits bei einer Konzentration von 150 mM ein, was bei Verwendung von Glucose als C-Quelle erst bei 90 mM der Fall war.

Experiment 2.5: Fermentation mit Glycerin als C-Quelle und höherer Substratkonzentration

In der Fermentation gemäß Experiment 2.4 konnte beobachtet werden, dass bereits bei einer Pseudocumolkonzentration von 240 mM ein Stopp der Akkumulation von 3,4-Dimethylbenzylalkohol in der Kulturflüssigkeit auftrat. Dadurch erschien es möglich, die Biotransformation mit einer höheren Pseudocumolkonzentration zu starten und eine höhere Endkonzentration von 3,4-Dimethylbenzaldehyd (DBAld) zu erzielen.

Die Glycerinkonzentration betrug am Anfang des Batchs 0.7% (v/v). Zu Beginn betrug das Zelltrockengewicht 4.4 g/l und erreichte am Ende des Fed-Batchs 25 g/l (Figur 14). Glycerin wurde zu Beginn des Fed-Batchs mit einer Rate von 3.7 g/h zugeführt. Die Rate wurde danach stufenweise erhöht. Es wurde bis zur 10. Stunden keine Essigsäure gebildet. Diese akkumulierte sich jedoch bis zum Experimentende bis zu einer Konzentration von 26.7 g/l im Medium.

Die Pseudocumolkonzentration (in der organischen Phase) betrug 5.3% (v/v). Zuerst konnte gleichzeitig eine Bildung von 3,4-Dimethylbenzylalkohol (DBAlk) und 3,4-Dimethylbenzaldehyd (DBAld) festgestellt werden. Ab einer Pseudocumolkonzentration von 300 mM bis 150 mM wurde vor allem DBAld gebildet und akkumuliert. Die 3,4-Dimethylbenzoesäurebildung (DBSäure) begann ab einer Pseudocumolkonzentration von 150 mM. Die Endkonzentration von DBAlk betrug 80 mM während die Endkonzentration von DBAld 212 mM betrug (Figur 15).

Die Enzymaktivitäten waren wie in der ersten Fermentation mit Glycerin hoch. Die spezifische Aktivität (1 μmol Gesamtprodukt (DBAlk, DBAld und DBSäure) welches in 1 Minute / g CDW gebildet wird) erreichte einen Wert von 46 U/g CDW. Die spezifische Bildungsrate von 3,4-Dimethylbenzaldehyd betrug nach 5 Stunden 22 U/g CDW. Die Aktivität konnte auch bei dieser Fermentation nur während des Biomassenwachstums gehalten werden.

Die Erhöhung der Substratkonzentration hat auf die Endkonzentration von 3,4-Dimethylbenzaldehyd keinen Einfluss, da die Aktivitätsdauer nicht über längere Zeit aufrecht erhalten werden kann. Die Konzentration von 3,4-Dimethylbenzylalkohol am Ende des Fed-Batchs war hoch.

Beispiel 3: Repetitiver Fed-Batch

Um die Aktivität der Zellen in Abwesenheit von Kanamycin über längere Zeit zu überprüfen, wurden zwei aufeinanderfolgende Fed-Batch Experimente durchgeführt, wobei 10 ml des ersten Fed-Batchs als Inokulum für den zweiten Fed-Batch dienten. Ziel war es die Stabilität des Plasmids und der XMO Gene über mehrere Generationen ohne Selektionsdruck zu untersuchen. Als C-Quelle wurde Glucose verwendet. Mit Start der Feedzufuhr wurde 4 ml der Thiaminstocklösung (1 % w/v) und 4 ml der Spurenelementlösung US* zugegeben.

1. Vorkultur: 4 ml LB-Medium (1 % Glucose (w/v), 4 μl Kanamycin (50 mg/ml)) mit frisch transformierten Zellen animpfen und bei 37 °C über Nacht inkubieren

2. Vorkultur: 100 ml M9*-Medium (0.5% Glucose (w/v), 200 μl Magnesiumsulfat, 100 μl Spurenelementlösung US*, 100 μl Thiamin (1 % w/v), 100 μl Kanamycin (50 mg/ml)) mit 4 ml 1. Vorkultur animpfen und bei 30°C 10 - 12 Stunden inkubieren.

900 ml Medium, welches im Bioreaktor enthalten sind werden mit 100 ml Vorkultur inokuliert. Nach dem Batch (9-12 h) wird der Fed-Batch gestartet indem eine Feedlösung kontinuierlich zugeführt wird. 1 Stunde späterwird dem Bioreaktor 11 organische Phase (DOP), in welcher das Substrat (P) und der Induktor (Octan) enthalten ist, zugegeben. Nach 15 Stunden wird die Kulturflüssigkeit bis auf ein Volumen von 10 ml, welches als Inokulum für den 2. Fed-Batch dient, abgeerntet. Dem Bioreaktor werden 990 ml Medium zugegeben. Abermals wird eine Biotrahsformation identisch wie der 1. Fed-Batch durchgeführt.

Experiment 3.1: Fed-Batch 1. Tag

Vor der Inokulation betrug die Glucosekonzentration 0.7% (w/v). Bei Start des Fed-Batchs wurde dem Medium zur Induktion der Zellen 0.1 % (v/v) Octan zugegeben. Vor dem Start des Fed-Batchs betrug die Biomasse 4.5 g/l und wuchs während des Fed-Batchs bis auf 28 g/l an (Figur 16). Die Glucosezufuhrrate betrug zu Beginn des Fed-Batchs 9 g/h und wurde anschliessend stufenweise erhöht. Die Essigsäurekonzentration betrug am Ende des Fed-Batchs 25 g/l.

Die Biotransformation wurden mit einer Substratkonzentration von 4% (v/v) gestartet. Die Konzentrationsverläufe von Pseudocumol, 3,4-Dimethylbenzylalkohol (DBAlk), 3,4-Dimethylbenzaldehyd (DBAld), 3,4-Dimethylbenzoesäure (DBSäure) und Octan sind in Figur 17 dargestellt. Es konnte zuerst eine 3,4-Dimethylbenzaldehyd und eine 3,4-Dimethylbenzylalkoholbildung festgestellt werden. Nach 6.5 Stunden nach Start des Fed-Batchs bei einer Substratkonzentration von 150 mM wurde vor allem DBAld gebildet. Die Säurebildung setzte nach 12.5 Stunden ein. Die 3,4-Dimethylbenzaldehydkonzentration betrug am Ende der Biotransformation 220 mM und jene vom DBAlk 14 mM. Das Pseudocumol konnte während dem Fed-Batch bis auf eine Konzentration von 13 mM verwertet werden.

3 Stunden nach Start des Fed-Batchs betrug die spezifischen Aktivität 27 U/g CDW. Die Aldehydbildungsrate erreichte nach 5 Stunden einen Höchstwert von 16 U/g CDW. Die Enzymaktivität konnte über 15 Stunden aufrecht erhalten werden.

Experiment 3.2: Fed-Batch 2. Tag

Die Glucosekonzentration betrug vor der Inokulation 0.7% (v/v). Die Biomasse betrug nach dem Batch 4.5 g/l und erreichte am Ende des Fed-Batchs eine Konzentration von 28 g/l. Die Glucosezufuhrrate betrug am Anfang 9 g/h, wurde jedoch bei einer zu starken Glucoselimitation erhöht. Die Essigsäurekonzentration betrug am Ende der Biotransformation 44 g/l (Figur 18).

1 Stunde nach Beginn des Fed-Batchs wurde dem Medium die organische Phase, in welcher eine Substratkonzentration von 4% (v/v) war, zugegeben. Die Konzentrationsverläufe von Pseudocumol, DBAld, DBAlk und DBSäure verliefen ähnlich wie im Fed-Batch 1. Tag (Experiment 3.1). Oberhalb einer Pseudocumolkonzentration von 150 mM konnte gleichzeitig eine DBAld- und eine DBAlk-Bildung festgestellt werden. Unterschritt die Substratkonzentrationen einen Wert von 150 mM wurde vor allem DBAld gebildet bis zu einer Endkonzentration von 220 mM. Die DBSäurebildung

setzte nach 12.5 Stunden ein. Die Endkonzentration von DBAlk betrug 30 mM und jene der DBSäure 13 mM (Figur 19).

Die spezifische Aktivität ereichte im 2. Fed-Batch einen identischen Höchstwert, wie jene vom Fed-Batch 1. Tag (Experiment 3.1) von 27 U/g CDW. Ebenso erreichte die Aldehydbildungsrate einen identischen Höchstwert von 16 U/g CDW. Die Enzymaktivität konnte über 15 Stunden aufrecht erhalten werden.

Die beiden Biotransformationen lieferten ähnliche, wenn nicht sogar identische Resultate. So konnte in beiden Fed-Batchs hohe spezifische Aktivitäten und Aldehydbildungsraten festgestellt werden. Ebenso konnten identische 3,4-Dimethylbenzaldehydkonzentrationen am Ende der beiden Biotransformationen erreicht werden. Die Enzymaktivität konnte bei beiden Fed-Batchs über 15 Stunden aufrecht erhalten werden. Die Stabilität des Plasmids war sehr hoch. So blieb über 60 Stunden und 14 Generationen das Plasmid auch ohne Selektionsdruck erhalten.

Es konnten keine signifikanten Unterschiede der Koloniezahlen auf den LB-Agarplatten und den LB-Agarplatten mit Kanamycin festgestellt werden.

II. VERSUCHE ZUR BIOTRANSFORMATION IM PILOTMASSSTAB

Die Biotransformation wurde nach Beendigung der Versuche im Labormasstab im Pilotmassstab (Beispiele 4, 5) durchgeführt. Dazu wurde ein 42-Liter Rührreaktor verwendet. Das Volumen des Ansatzes, welcher zu gleichen Teilen aus Riesenbergmedium und organischer Phase mit einer Substratkonzentration von 4.3 % (v/v) bestand, betrug 30 Liter. Vor der Inokulation wurde dem Medium 0.7% (w/v) Glucose zugegeben. Auf die Zugabe von Kanamycin wurde wie schon im repetitiven Fed-Batch verzichtet.

Beispiel 4: Fermentation im Pilotmassstab bei pH 6.6

Vor dem Start des Fed-Batchs betrug das Zelltrockengewicht 4 g/l. Die Endkonzentration betrug jedoch nur 14.5 g/l. 2 Stunden nach der Induktion wies der pH-Wert aufgrund eines Fehlers bei der pH-Regelung, nur noch einen Wert von 6.6 auf, anstelle von 7.1. Zu diesem Zeitpunkt war ein Wachstumsstopp der Zellen zu beobachten. Die Rate der Glucosezufuhr betrug 135 g/h (w/v). Die Zellen waren während der Biotransformation nicht Glucoselimitiert. Die Essigsäurekonzentration nahm stetig zu bis zu einer Endkonzentration von 47 g/l (Figur 20).

Die Substratkonzentration in den 15 l organischer Phase betrug 4.3% (v/v). Die 3,4-Dimethylbenzylalkoholbildung (DBAlk) setzte 2 Stunden nach Induktion der Zellen ein (Figur 21). Die 3,4-Dimethylbenzaldehydbildung begann erst4 Stunden nach der Induktion der Zellen. Nach 26 Stunden betrug die Konzentration von 3,4-Dimethylbenzaldehyd 130mM und diejenige von 3,4-Dimethylbenzylalkohol 100 mM. Die 3,4-Dimethylbenzoesäurebildung war sehr gering. So betrug die Endkonzentration lediglich 2 mM.

Der niedrige pH-Wert beeinflusste nicht nur das Zellwachstum sondern auch die Enzymaktivität. Die spezifische Aldehydbildungsrate war am Anfang der Biotransformation wegen der pH-Reduktion tief. Nach 4 Stunden bei pH 7,1 erholte sie sich jedoch wieder und erreichte einen Wert von 12 U/g CDW. Die spezifische Gesamtaktivität erreichte erst nach 7 Stunden nach Beginn des Fed-Batchs den Höchstwert von 32 U/g CDW.

Die vorübergehende pH-Erniedrigung hatte einen starken Einfluss auf das Biomassenwachstum und die Enzymaktivität. Im Vergleich zu vorhergegangenen Fermentationen erreichte die Biomasse eine tiefe Konzentration. Die volumetrische Aktivität (U/l) war während der Biotransformation dementsprechend tief.

Die Kinetik des Mehrstufenprozesses war jedoch dieselbe wie in den Biotransformationen im Labormassstab. So bildete sich bis zu einer Pseudocumolkonzentration von 150 mM auch 3,4-Dimethylbenzylalkohol.

Beispiel 5: Fermentation im Pilotmassstab bei pH 7.4

Da die vorhergegangene Fermentation nicht die erwartete Ausbeute von 3,4-Dimethylbenzaldehyd ergab, wurde das Experiment wiederholt. Während der Biotransformation wurde der pH-Wert auf 7.4 erhöht. Somit war es auch möglich den Einfluss eines erhöhten pH-Werts auf die Biotransformation zu testen.

Nach dem Batch betrug das Zelltrockengewicht 3.5 g/l. Die Zelldichte nahm während des Fed-Batchs bis auf einen Wert von 20 g/l zu. Da die Glucose im Medium nie limitierend war nahm die Essigsäurekonzentration im Medium kontinuierlich zu (Figur 22). Die Essigsäurekonzentration nach der Biotransformation betrug 45 g/l. Bereits eine Stunde nach der Zugabe der organischen Phase wurde der Zuluft Sauerstoff beigemischt um eine Sauerstofflimitation zu verhindern. Der DOT wurde bis zur 6. Stunde der Biotransformation auf einen Wert zwischen 15% - 25% gehalten. Danach betrug der Gehalt an gelöstem Sauerstoff im Medium zwischen 25%- 40%.

Die Konzentrationsverläufe während der Biotransformation von Pseudocumol, 3,4-Dimethylbenzylalkohol (DBAlk), 3,4-Dimethylbenzaldehyd (DBAld) und 3,4-Dimethylbenzoesäure (DBSäure) können mit den Verläufen von den Fermentationen im Labormassstab verglichen werden. Bei einer Pseudocumolkonzentration von 150 mM wird vor allem 3,4-Dimethylbenzaldehyd in der Kulturflüssigkeit akkumuliert. Ausgehend von einer Substratkonzentration von 4.8% (v/v) betrug die Konzentration von Pseudocumol am Ende der Fermentation 3 mM. Die Endkonzentration von

DBAlk betrug 4 mM und jene der DBSäure 12 mM. Die 3,4-Dimethylbenzoe-säurebildung setzte erst nach ca. 9 Stunden ein. DBAld wurde während der Biotransformation bis zu einer Endkonzentration von 275 mM produziert. Das zugegebene Substrat konnte zu 78.6% in DBAld umgewandelt werden (Figur 23). Jedoch wurde während des Fed-Batchs wegen der Belüftung einen Verlust von 16% Pseudocumol verzeichnet. So waren am Ende beim Abernten des Reaktors die in Tabelle 3 aufgeführten prozentualen Anteile in der organischen Phase enthalten.

Tabelle 3: Prozentualer Anteil von Pseudocumol, DBAlk, DBAld und DBSäure in der organischen Phase nach der Biotransformation

| Substanz | Anteil in [%] der organischen Phase |
|---|---|
| Pseudocumol | 1 |
| DBAlk | 1.3 |
| DBAld | 93.6 |
| DBSäure | 4.1 |

Die hohen Werte der spezifischen Aktivität (51 U/g CDW) sowie der Aldehydbildungsrate (30 U/g CDW) wurden durch die Erhöhung des pH-Werts von 7.1 auf 7.4 drei Stunden nach der Zugabe der organischen Phase erreicht. In Figur 24 sind die Verläufe der spezifischen Gesamtaktivität und der Aldehydbildungsrate graphisch dargestellt. So konnte bei der Erhöhung des pH-Werts nach 3 Stunden ein deutlicher Anstieg der spezifischen Aktivität und der spezifischen Aldehydbildungsrate verzeichnet werden.

Das zugegebene Substrat wurde bei nur geringer Bildung der Nebenprodukte 3,4-Dimethylbenzylalkohol und 3,4-Dimethylbenzoesäure zu einem hohen Prozentsatz in 3,4-Dimethylbenzaldehyd umgeformt. Die Erhöhung des pH-Werts hatte einen positiven Einfluss auf die Enzymaktivität. So konnten nach der Erhöhung deutlich höhere Werte der spezifischen Aktivität und der Aldehydbildungsrate erzielt werden.

Der im Labormassstab entwickelte zweiphasige Fed-Batch-Prozess liess sich mit Erfolg auf den Pilotmassstab übertragen.

Beispiel 6: Aufarbeitung von 3,4-Dimethylbenzaldehyd von Fermentation im Pilotmassstab bei pH 7.4

Nach Beendigung der Biotransformation im Pilotmassstab wurde der Bioreaktor abgeerntet. Die wässrige und die organische Phase wurden durch Zentrifugieren separiert. Anschliessend wurde die organische Phase durch die Zugabe von Natriumsulfat getrocknet und danach getrennt. Um das 3,4-Dimethylbenzaldehyd vom Dioctylphthalat trennen zu können, wurde eine Feinvakuumdestillation eingesetzt. Das Vakuum wurde zwischen 0.08 mbar und 0.15 mbar geregelt. Bei einer Temperatur des Dioctylphthalat von 110°C - 120°C begann das 3,4-Dimethylbenzaldehyd zu destillieren.

Es konnte 484 ml Destillat während der Destillation aus 14 Liter Dioctylphthalat gewonnen werden. Das Destillat bestand zu 96.5% aus 3,4-Dimethylbenzaldehyd, 0.3% aus 3,4-Dimethylbenzylalkohol, 2.8% aus Dioctylphthalat, 0.3% aus Pseudocumol und 0.04% aus Octan.

ZUSAMMENFASSUNG DER EXPERIMENTELLEN ERGEBNISSE

1. Optimierung prozessrelevanter Parameter

1.1 Der Einfluss von Sauerstoff und Glucose

Ein wichtiger Parameter bei der Produktion von 3,4-Dimethylbenzaldehyd mittels eines biokatalytischen Prozesses ist die Aktivität des Enzyms. Dabei ist die Höhe und die Dauer der Enzymaktivität von entscheidender Bedeutung.

Wie gezeigt wurde, haben die Glucosekonzentration und die Sauerstoffversorgung der Zellen einen Einfluss auf die Enzymaktivität. So hat eine Glucosekonzentration über 10 g/l im Medium eine inhibierende Wirkung auf die Enzymaktivität. Eine zu starke Glucoselimitation hat jedoch auch eine negative Wirkung auf die Produktivität des Katalysators.

In dieser Arbeit wurde gezeigt, dass bei einer gleichzeitigen Glucose- und Sauerstofflimitation die Xylomonooxygenase die Katalyse einstellt und unspezifische Alkoholdehydrogenasen von *E. coli* 3,4-Dimethylbenzaldehyd wieder in 3,4-Dimethylbenzylalkohol zurückbilden (Figur 6).

Wenn Sauerstoff- oder Glucoselimitation einzeln auftraten und nicht zu stark waren, wurden keine inhibierenden Einflüsse auf die Aktivität beobachtet (Figuren 16 und 18).

1.2 Der Einfluss der Substratkonzentration

Die Substratkonzentration hat ebenfalls einen Einfluss auf die Enzymaktivität und bei einer Pseudocumolkonzentration unter 150 mM in der organischen Phase auch auf die Bildung von 3,4-Dimethylbenzaldehyd. So hat eine niedrige Pseudocumolkonzentration eine niedrige Enzymaktivität zur Folge. Bei einer Pseudocumolkonzentration über 150 mM ist nur der erste Schritt des Mehrstufenprozesses, die Bildung des 3,4-Dimethylbenzylalkohols (DBAlk) , von der Substratkonzentration abhängig.

Bei einer anfänglichen Pseudocumolkonzentration von weniger als 150 mM konnte vor allem eine Bildung von 3,4-Dimethylbenzaldehyd (DBAld) beobachtet werden. Daher wurde kein 3,4-Dimethylbenzylalkohol in der Kulturflüssigkeit angereichert (Figur 3). Aus diesem Grund wurde ab einer Pseudocumolkonzentration von 90 mM vermehrt 3,4-Dimethylbenzoesäure (DBSäure) gebildet. Die später eintretende 3,4-Dimethylbenzylalkoholbildung kann auf die zu diesem Zeitpunkt sehr geringe Enzymaktivität zurückgeführt werden.

War die Anfangskonzentration jedoch höher als 150 mM, wurde zuerst eine gleichzeitige 3,4-Dimethylbenzylalkohol- und 3,4-Dimethylbenzaldehydakkumulation in der organischen Phase beobachtet. Unterschritt die Pseudocumolkonzentration 150 mM, so wurde vor allem DBAld gebildet. Die Bildung von DBSäure setzte am Ende der Biotransformation ein, als der DBAlk praktisch vollständig in DBAld umgesetzt wurde (Figuren 11, 23). Dies bestätigt die inhibierende Wirkung von DBAlk auf die 3,4-Dimethylbenzoesäurebildung.

Noch höhere Anfangskonzentrationen von Pseudocumol können eine zu hohe 3,4-Dimethylbenzylalkoholanreicherung in der wässrigen Phase zur Folge haben. Übersteigt die 3,4-Dimethylbenzylalkoholkonzentration in der wässrigen Phase einen Wert von 100 mM, wirkt er auf die Zellen toxisch.

1.3 Einfluss der C-Quelle

Obwohl die Aktivitäten bei Verwendung von Glycerin als C-Quelle um einiges höher waren, wurde schliesslich nicht mehr Produkt gebildet (Figur 12 und Figur 15). In der organischen Phase akkumulierten ausserdem höhere Konzentrationen,an Nebenprodukten. Dies kann unter anderem der kürzeren Aktivitätsdauer zugeschrieben werden. So konnte lediglich während des Biomassenwachstums eine Enzymaktivität beobachtet werden. Wurde die stationäre Phase erreicht, war ein sofortiger Rückgang der Aktivität zu beobachten. Bei Glucose als C-Quelle konnte dagegen eine Enzymaktivität bis in die stationäre Phase beobachtet werden. Ausserdem wurde beobachtet, dass der gebildete 3,4-Dimethylbenzylalkohol bei Verwendung von Glycerin als C-Quelle die 3,4-Dimethylbenzoesäurebildung in einem geringeren Masse inhibiert als bei Glucose als C-Quelle der Fall war (Figur 15).

1.4 Der Einfluss des pH-Wertes

Eine Erhöhung des pH-Werts hat auf die Enzymaktivität einen positiven Einfluss. So konnte bei der Fermentation mit einem pH von 7.4 anstelle von 7.1 eine deutliche Steigerung der spezifischen Aktivität sowie der Aldehydbildungsrate erzielt werden (Figur 24). Wurde der pH bei Fermentationen, in denen Glucose als C-Quelle verwendet wurde, auf 7.1 eingestellt, betrug die Aldehydbildungsrate maximal 16 U/g CDW. Bei pH 7.4 wurde eine deutlich höhere Aldehydbildungsrate von 30 U/g CDW erreicht. Ein pH von 6.6 hat eine deutlich negative Auswirkung auf die Enzymaktivität (Produktivität).

2. Plasmidstabilität

3,4-Dimethylbenzaldehyd wird erfindungsgemäß durch rekombinante *E.coli* JM101 (pSZP3) produziert. Das Plasmid pSZP3 wurde von Panke (1999) entwickelt. Um zu gewährleisten, dass das Plasmid in den Bakterien erhalten bleibt, trägt es ein Resistenzgen welches zur Resistenz gegen das Antibiotikum Kanamycin führt. In grosstechnischen Prozessen ist die Zugabe von Antibiotikum aus preislichen und umwelttechnischen Gründen nicht wünschenswert. Aus diesem Grund wurden Versuche durchgeführt, in welchen die Plasmidstabilität ohne Selektionsdruck durch Kanamycin getestet wurde. Wie durch den repetitiven Batch gezeigt werden konnte, bleibt das Plasmid und die XMO-Gene über 60 Stunden stabil. Das heisst über 14 Generationen wird das Plasmid erhalten und die XMO-Gene werden exprimiert.

3. Optimierung des Mediums

Soll ein Bioprozess industriell genutzten werden, müssen einige Voraussetzungen an das Medium erfüllt sein. So sollte das Medium möglichst definiert sein. Das heisst es sollte keine Inhaltsstoffe enthalten, deren Konzentrationen nicht genau bestimmt werden können und die die Reinheit des Produkts gefährden können. So ist, wenn möglich, auf undefinierte Inhaltsstoffe, wie Hefeextrakt oder Pepton, zu verzichten.

Das Erreichen hoher Zelldichten bei Bioprozessen hat auf die Rentabilität des Prozesses grossen Einfluss. So

können einem Hochzelldichteprozess folgende Vorteile zugeschrieben werden: kleineres Reaktorvolumen, weniger Aufwand im Vorbereitungs- und Aufarbeitungsprozess und eine wesentlich höhere volumetrische Aktivität. Jedoch ist ein Prozess mit hohen Zelldichten auch mit Problemen behaftet. Es müssen hohe Mengen an Nährstoffen zugegeben werden und durch die hohe Zelldichte ist die Sauerstoffversorgung der Zellen problematisch. Ausserdem können Hochzelldichtekultivationen durch metabolische Nebenprodukte wie, Ethanol, Acetat und Laktat, inhibiert werden, die zum Beispiel unter aneoroben Bedingungen gebildet werden. Auch unter aeroben Kulturbedingungen können von *E. coli* Säuren und andere metabolische Nebenprodukte gebildet und im Medium akkumuliert werden. Essigsäure hat bei einem pH von 7 bereits bei einer Konzentration von 5 g/l einen inhibitorische Wirkung auf das Zellwachstum. Daher sollte in einem Bioprozess eine zu starke Säurebildung verhindert werden.

Riesenberg (1991) entwickelte ein definiertes Medium, in welchem Glucose und Magnesiumsulfat nicht im Anfangsmedium enthalten ist. Es zeichnet sich unter unlimitierten aeroben Kulturbedingungen durch geringe Essigsäurebildung aus. Unter Verwendung dieses Mediums konnten mit *E. coli* schon Zelldichten von 110 g/l erreicht werden. (Riesenberg 1991).

<u>3.1 Der Einfluss des Riesenbergmediums</u>

Da bei Verwendung des M9 Mediums nur mit Hefeextrakt in der Feedlösung Zelldichten von 20-27 g/l erreicht wurden, wurde die Biotransformation mit dem Riesenbergmedium durchgeführt. Wie in Figur 8 gezeigt ist, kann mit einer entsprechenden Glucosekonzentration eine hohe Zelldichte im Batch erreicht werden. Die Essigsäurebildung war identisch zu Konzentrationen, die von Riesenberg, Schulz et al. (1991) erreicht wurde. So wurde ca. 1 g/l Essigsäure gebildet. Ausgehend von einem Zelltrockengewicht von 16 g/l wurde eine Biotransformation durchgeführt. Trotz genügend vorhandenem Sauerstoff wurde eine starke Essigsäurebildung festgestellt, was sich schliesslich auf das Biomassenwachstum auswirkte. So wurde lediglich ein Zelltrockengewicht von 24.7 g/l erreicht. (Figur 8). Die Bildung von Essigsäure konnte schon bei geringen Glucosekonzentrationen beobachtet werden (Figur 4). Dies könnte auf die Bildung von Xylolmonooxygenase zurückzuführen sein, da diese das Wachstum von *E. coli* hemmt (Bühler, Schmid et al. 2000).

Wenn anstelle von Glucose als C-Quelle Glycerin verwendet wurde, akkumulierte weniger Essigsäure in der wässrigen Phase (Figuren 10 und 12).

Die Sauerstoffversorgung der Zellen in einer Hochzelldichtekultivation ist problematisch. Hinzu kommt die Tatsache, dass das für die Biotransformation verwendete Enzym, die Xylolmonooxygenase, für die Katalyse der Substanzen Sauerstoff benötigt (Bühler, Schmid et al. 2000). Bereits bei dem in den Fermentationen erreichten Zelltrokkengewicht von unter 30 g/l war das Reaktorsystem im Bezug auf die Rührgeschwindigkeit und der Belüftung völlig ausgelastet. Deshalb musste im Pilotmassstab ein Sauerstoff-Luftgemisch zur Belüftung verwendet werden, um die Zellen mit genügend Sauerstoff versorgen zu können.

<u>3.2 Feedzusammensetzung</u>

Das Riesenbergmedium zeichnet sich gegenüber dem M9 Medium durch eine kostengünstigere Mediumzusammensetzung aus. Zudem konnte auf das Hefeextrakt in der Feedlösung verzichtet und die damit verbundenen Probleme während des Prozesses bei der Produktaufreinigung (Schaumbildung, Phasentrennung).

Wurde bei Verwendung des M9 Mediums auf Hefeextrakt in der Feedlösung verzichtet, konnten negative Einflüsse auf das Biomassenwachstum festgestellt werden. So betrug das Zelltrockengewicht am Ende des Fed-Batchs lediglich 12 g/l (Figur 4). Im Gegensatz dazu konnte mit dem Riesenbergmedium Zelldichten von 25 - 30 g/l erzielt werden. Das geringere Biomassenwachstum bei Einsatz vom M9 Medium könnte auf die geringere Ammoniumkonzentration gegenüber dem Riesenbergmedium zurückgeführt werden.

<u>4. Vernetzter Einfluss verschiedener Parameter</u>

Im Rahmen der Erfindung konnten prozessrelevante Parameter bestimmt und verbessert werden. In Figur 25 sind die verschiedenen Parameter und deren Einflüsse auf den Gesamtprozess schematisch dargestellt.

Wie schon beschrieben haben der gelöste Sauerstoff und die Glucosekonzentration im Medium einen erheblichen Einfluss auf die Aktivitätshöhe und Aktivitätsdauer. Die Substrat und Produktkonzentrationen haben je nach Substanz eine toxische Wirkung auf die Biomasse. So inhibiert die DBSäure das Biomassenwachstum bereits schon bei einer Konzentration von 16 mM in der wässrigen Phase. Die auf das Biomassenwachstum inhibierende Konzentration von DBAlk liegt bei einer Konzentration von 100 mM in der wässrigen Phase und jene vom DBAld bei 1812 mM. Übersteigt die Pseudocumol eine Konzentration von 15 - 25% in der wässrigen Phase wirkt es ebenfalls toxisch auf die Zellen. Ebenso hat die Essigsäurekonzentration einen inhibierenden Einfluss auf das Biomassenwachstum.

## 5. Produktaufreinigung

### 5.1 Phasentrennung

Der erste Schritt nach dem Ernten des Reaktors ist die Phasenauftrennung durch Zentrifugieren. Es konnten wie schon erwähnt durch die Zugabe von Hefeextrakt und Glycerin ins Medium negative Auswirkungen auf die Trennung festgestellt werden. So hat sich eine Zwischenphase gebildet, die bis zu einem Drittel des aufzutrennenden Volumens ausmachte. Ein vergleichbarer Effekt konnte beobachtet werden, wenn die Rührgeschwindigkeit mehr als 2000 rpm betrug. Zurückzuführen ist dieses Phänomen auf Proteine, die im Hefeextrakt vorhanden sind oder auf die, durch zu hohe Rührgeschwindigkeit verursachte, Zerstörung der Zellen.

### 5.2 Destillation

3,4-Dimethylbenzaldehyd ist eine hochsiedende Substanz. Die Siedetemperatur liegt bei Normaldruck bei 220-223°C. Die erfindungsgemäß verwendete organische Phase Dioctylphthalat weist eine Siedetemperatur von 380°C auf. Dieser grosse Siedetemperaturunterschied der beiden Substanzen ermöglichte es 3,4-Dimethylbenzaldehyd durch Destillation zu reinigen.

Um das 3,4-Dimethylbenzaldehyd aus der organischen Phase abzudestillieren, müsste die Lösung um einiges höher als 223°C erhitzt werden. Aus diesem Grund erschien eine Feinvakuumdestillation als geeignete Methode.

## 6. Biotransformation im Pilotmassstab

Wie erfindungsgemäß gezeigt werden konnte, ist ein up-scaling des zur Produktion von 3,4-Dimethylbenzaldehyd entwickelten zweiphasigen Fed-Batch-Prozesses ohne grössere Probleme möglich. Nachdem im Labormassstab die prozessrelevanten Parameter und das Medium optimiert wurden, wurde der Prozess auf den Pilotmassstab übertragen. Als problematisch erwies sich während der Biotransformation einzig die Sauerstoffversorgung der Zellen. So musste der Zuluft bereits 2 Stunden nach der Induktion der Zellen Sauerstoff beigemischt werden.

## LITERATURVERZEICHNIS

F. Ausubel et al.,(1997) Current Protocols in Molecular Biology, Wiley Interscience, New York

Bühler, B., A. Schmid, et al. (2000). "Xylene monooxygenase catalyzes the multistep oxygenation of toluene and pseudocumene to corresponding alcohols, aldehydes, and acids in Escherichia coli JM101." The Journal of Biological Chemistry 275(14): 10085-10092.

Harayama, S., M. Kok, et al. (1992). "Functional and evolutionary relationships among diverse oxygenases." Ann. Rev. Microbiol. 46: 565-601.

Harayama, S., R. A. Leppik, et al. (1986). "Gene order of the TOL catabolic plasmid upper pathway operon and oxidation of both toluene and benzyl alcohol by the xylA product." Journal of Bacteriology 167(2): 455-461.

Harayama, S., M. Rekik, et al. (1989). "Characterization of five genes in the upperpathway operon of TOL plasmid pWW0 from Pseudomonas putida and identification of the gene products." Journal of Bacteriology 171(9): 5048-5055.

Legoy, M. D., H. S. Kim, et al. (1985). "Use of alcohol dehydrogenase for flavor aldehyde production." Process Biochemistry 20: 145-148.

Molinari, F., F. Aragozzini, et al. (1997). "Continuous production of isovaleraldehyde through extractive bioconversion in a hollow-fiber membrane bioreactor." Enzyme and Microbial Technology 20: 604-611.

Molinari, F., R. Gandolfi, et al. (1999). "Biotransformations in two-liquid-phase systems. Production of phenylacetaldehyde by oxidation of 2-phenylethanol with acetic acid bacteria." Enzyme and Microbial Technology 25: 729-735.

Molinari, F., R. Villa, et al. (1995). "Aldehyde production by alcohol oxidation with Gluconobacter oxydans." Applied Microbiology and Biotechnology 43: 989-994.

Panke, S. (1999). Production of (S)-styrene oxide with recombinant bacteria. Institute of Biotechnolgy. Zurich, Swiss Federal Institute of Technology: 208.

Pouwels P. H. et al, (1985) "Cloning Vectors", Hrsg, Elsevier, Amsterdam-New York-Oxford

Ramos, J. L., S. Marqués, et al. (1997). "Transcriptional control of the Pseudomonas TOL plasmid catabolic operons is achieved through an interplay of host factors and plasmid-encoded regulators." Annual Reviews in Microbiology 51: 341-373.

Riesenberg, D. (1991). "High-cell-density cultivation of Escherichia coli." Current Opinion in Biotechnology 2: 380-384.

Sambrook, J., E. F. Fritsch, et al. (1989). Molecular cloning: a laboratory manual, 2nd ed. Cold Spring Harbor,

N.Y., Cold Spring Harbor Laboratory.
Schmid, A. (1997). Der Metabolismus von ortho-Hydroxybiphenylen in Pseudomonas azeleica HBP1, Universität Stuttgart.
Simmonds, J. and G. K. Robinson (1997). "Novel biotransformations to produce aromatic and heterocyclic aldehydes." Enzyme and Microbial Technology 21: 367-374.
Simmonds, J. and G. K. Robinson (1998). "Formation of benzaldehyde by Pseudomonas putida ATCC 12633." Applied Microbiology and Biotechnology 50: 353-358. Williams, P. A., L. M. Shaw, et al. (1997). "xy1UW, two genes at the start of the upper pathway operon of TOL plasmid pWW0, appear to play no essential part in determining its catabolic potential." Microbiology 143: 101-107.
Wittcoff, H. A. and B. G. Reuben (1996). Industrial Organic Chemicals. New-York, John Wiley & Sons . Inc.

## Patentansprüche

1. Verfahren zur ein- oder mehrstufigen biokatalytischen Oxidation aromatischer Verbindungen wobei man

   a) in einem zweiphasigen, wässrig-organischen Kulturmedium, welches ein aromatisches Substrat der Formel II

   $$Ar\text{-}R^2 \qquad (II)$$

   worin
   Ar für einen gegebenenfalls ein- oder mehrfach substituierten einkemigen aromatischen Ring steht; und
   $R^2$ für $-(CH_2)_{n+1}R^3$ steht, worin
   n für eine ganzzahligen Wert von 0 bis 15 steht; und
   $R^3$ für H steht;
   enthält, einen Mikroorganismus unter aeroben Bedingungen kultiviert, der ein Enzym mit Xylolmonooxygenase (XMO)-Aktivität exprimiert; und
   b) wenigstens ein bei der Oxidationsreaktion gebildetes Produkt, worin der Rest $R^2$ zum korrespondierenden Hydroxy-, Carbonyl- oder Carboxyl-Rest oxidiert wurde, aus dem Kulturmedium isoliert;
   und wobei der Oxidationsgrad des eingesetzten Substrats über die Einstellung der Substratkonzentration steuerbar ist.

2. Verfahren nach Anspruch 1, **dadurch gekennzeichnet, dass** für die organische Phase eine apolare organische Verbindung verwendet wird, die in einem n-Octanol/Wasser-Zweiphasensystem einen Verteilungskoeffizienten von $>10^4$ aufweist.

3. Verfahren nach einem der vorhergehenden Ansprüche, **dadurch gekennzeichnet, dass** die organische Phase bei 1 atm eine Siedepunkt oder Siedebereich aufweist, der um etwa 50 bis 200 °C über dem Siedepunkt des Oxidationsprodukts (der Oxidationsprodukte) liegt.

4. Verfahren nach einem der vorhergehenden Ansprüche, **dadurch gekennzeichnet, dass** als organische Phase Di-($C_5$-$C_{12}$-alkyl)-phthalat oder ein Gemisch solcher Phthalate verwendet wird.

5. Verfahren nach einem der vorhergehenden Ansprüche, **dadurch gekennzeichnet, dass** man die organische Phase abtrennt und das (die) darin gelöste(n) Oxidationsprodukt(e) abdestilliert.

6. Verfahren nach einem der vorhergehenden Ansprüche, **dadurch gekennzeichnet, dass** der XMO-Aktivität exprimierende Mikroorganismus im wesentlichen keine Benzylalkoholdehydrogenase (BADH) und/oder keine Benzaldehyddehydrogenase (BZDH)-Aktivität besitzt.

7. Verfahren nach einem der vorherigen Ansprüche, **dadurch gekennzeichnet, dass** man einen rekombinanten Mikroorganismus verwendet, welcher mit einem Expressionsvektor transformiert ist, der unter der genetischen Kontrolle des regulativen alk-Systems aus Pseudomonas oleovorans GPo1 die für XMO kodierenden Gene xylM und xylA in operativer Verknüpfung enthält.

8. Verfahren nach Anspruch 7, **dadurch gekennzeichnet, dass** der Mikroorganismus mit dem Expressionsplasmid pSPZ3 transformiert ist.

9. Verfahren nach einem der vorhergehenden Ansprüche, **dadurch gekennzeichnet, dass** der Mikroorganismus ein Bakterium der Gattung Escherichia ist.

10. Verfahren nach einem der vorhergehenden Ansprüche, **dadurch gekennzeichnet, dass** man die Enzymexpression durch Zugabe eines Induktors zum Kulturmedium startet.

11. Verfahren nach einem der vorhergehenden Ansprüche, **dadurch gekennzeichnet, dass** man Xylolmonoxygenase aus *Pseudomonas putida* mt-2 exprimiert.

12. Verfahren nach einem der vorhergehenden Ansprüche, **dadurch gekennzeichnet, dass** man ein Reaktionsmedium verwendet, welches im wesentlichen kein Antibiotikum enthält.

13. Verfahren nach einem der vorhergehenden Ansprüche, **dadurch gekennzeichnet, dass** man die Umsetzung in semi-kontinuierlicher BatchFahrweise durchführt.

14. Verfahren nach einem der vorhergehenden Ansprüche, **dadurch gekennzeichnet, dass** man als Edukt Pseudocumol einsetzt.

15. Verfahren nach einem der vorhergehenden Ansprüche, **dadurch gekennzeichnet, dass** man den pH-Wert des Reaktionsmediums als Regelgröße für die Produktivität des Biokatalysators benutzt.

## Claims

1. A process for the single- or multistep biocatalytic oxidation of aromatic compounds, which comprises

   a) growing, under aerobic conditions, a microorganism which expresses an enzyme with xylene monooxygenase (XMO) activity in a biphase aqueous-organic culture medium comprising an aromatic substrate of the formula II

   $$Ar\text{-}R^2 \qquad (II)$$

   in which
   Ar is an unsubstituted or mono- or polysubstituted mononuclear aromatic ring; and
   $R^2$ is $-(CH_2)_{n+1}R^3$ where
   n is an integer from 0 to 15; and
   $R^3$ is H;
   and
   b) isolating, from the culture medium, at least one product formed in the oxidation reaction, in which the radical $R^2$ has been oxidized to the corresponding hydroxyl, carbonyl or carboxyl radical;

   and it being possible to control the degree of oxidation of the substrate used by adjusting the substrate concentration.

2. The process according to claim 1, wherein the organic phase used is an apolar organic compound with a partition coefficient of $>10^4$ in a biphasic n-octanol/water system.

3. The process according to any of the preceding claims, wherein the organic phase has a boiling point or boiling range at 1 atm which exceeds the boiling point of the oxidation product(s) by approximately 50 to 200°C.

4. The process according to any of the preceding claims, wherein the organic phase used is di($C_5$-$C_{12}$-alkyl) phthalate or a mixture of such phthalates.

5. The process according to any of the preceding claims, wherein the organic phase is separated off and the oxidation product(s) dissolved therein is/are removed by distillation.

6. The process according to any of the preceding claims, wherein the microorganism which expresses XMO activity has essentially no benzyl alcohol dehydrogenase (BADH) activity and/or no benzaldehyde dehydrogenase (BZDH) activity.

7. The process according to any of the preceding claims, wherein a recombinant microorganism is used which is transformed with an expression vector containing the XMO-encoding genes xylM and xylA in operable linkage under the genetic control of the regulatory alk system from Pseudomonas oleovorans GPo1.

8. The process according to claim 7, wherein the microorganism is transformed with the expression plasmid pSPZ3.

9. The process according to any of the preceding claims, wherein the microorganism is a bacterium from the genus Escherichia.

10. The process according to any of the preceding claims, wherein enzyme expression is commenced by adding an inductor to the culture medium.

11. The process according to any of the preceding claims, wherein Pseudomonas putida mt-2 xylene monooxygenase is expressed.

12. The process according to any of the preceding claims, wherein a reaction medium is used which comprises essentially no antibiotic.

13. The process according to any of the preceding claims, wherein the conversion is carried out as a semicontinuous batch operation.

14. The process according to any of the preceding claims, wherein pseudocumene is used as starting material.

15. The process according to any of the preceding claims, wherein the pH of the reaction medium is used as the parameter for the productivity of the biocatalyst

**Revendications**

1. Procédé pour l'oxydation biocatalytique en une ou plusieurs étapes de composés aromatiques, dans lequel :

a) on met en culture dans des conditions aérobies dans un milieu de culture biphasique aqueux-organique, qui contient un substrat aromatique de formule II :

$Ar-R^2$ (II)

dans laquelle :

Ar représente un cycle aromatique à un seul noyau éventuellement substitué une ou plusieurs fois ; et
$R^2$ représente $-(CH_2)_{n+1}R^3$, où
n représente un nombre entier de 0 à 15 ; et
$R^3$ représente H ;
un micro-organisme qui exprime une enzyme ayant une activité de xylène mono-oxygénase (XMO) ; et

b) on isole du milieu de culture au moins un produit formé lors de la réaction d'oxydation, le radical $R^2$ ayant été oxydé pour donner le radical hydroxyle, carbonyle ou carboxyle correspondant ;
et dans lequel le degré d'oxydation du substrat utilisé peut être contrôlé en réglant la concentration de substrat.

2. Procédé selon la revendication 1, **caractérisé en ce que** l'on utilise, pour la phase organique, un composé organique apolaire qui présente dans un système biphasique du type n-octanol/eau, un coefficient de partage > $10^4$.

3. Procédé selon l'une quelconque des revendications précédentes, **caractérisé en ce que** la phase organique présente, sous 1 atm, un point d'ébullition ou une plage d'ébullition qui se situe environ 50 à 200 °C au-dessus du point d'ébullition du produit d'oxydation (des produits d'oxydation).

4. Procédé selon l'une quelconque des revendications précédentes, **caractérisé en ce que** l'on utilise, comme phase organique, du di (alkyle en $C_5$-$C_{12}$)-phtalate ou un mélange de ce type de phtalates.

5. Procédé selon l'une quelconque des revendications précédentes, **caractérisé en ce que** l'on sépare la phase organique, et **en ce que** l'on extrait par distillation le(s) produit(s) d'oxydation qui y sont dissous.

6. Procédé selon l'une quelconque des revendications précédentes, **caractérisé en ce que** le micro-organisme exprimant une activité de XMO ne présente sensiblement pas d'activité de benzyl-alcool déshydrogénase (BADH) et/ou d'activité de benzaldéhyde déshydrogénase (BZDH).

7. Procédé selon l'une quelconque des revendications précédentes, **caractérisé en ce que** l'on utilise un micro-organisme recombinant, qui est transformé avec un vecteur d'expression, lequel contient, sous le contrôle génétique du système de régulation alk tiré de l'espèce Pseudomonas oleovorans GPo1, les gènes xylM et xylA codant pour la XMO, reliés de manière opérationnelle.

8. Procédé selon la revendication 7, **caractérisé en ce que** le micro-organisme est transformé avec le plasmide d'expression pSPZ3.

9. Procédé selon l'une quelconque des revendications précédentes, **caractérisé en ce que** le micro-organisme est une bactérie de la souche Escherichia.

10. Procédé selon l'une quelconque des revendications précédentes, **caractérisé en ce que** l'on déclenche l'expression de l'enzyme en ajoutant un inducteur au milieu de culture.

11. Procédé selon l'une quelconque des revendications précédentes, **caractérisé en ce que** l'on exprime la xylène mono-oxygénase à partir de la souche Pseudomonas putida mt-2.

12. Procédé selon l'une quelconque des revendications précédentes, **caractérisé en ce que** l'on utilise un milieu réactionnel qui ne contient sensiblement pas d'antibiotique.

13. Procédé selon l'une quelconque des revendications précédentes, **caractérisé en ce que** l'on met en oeuvre la réaction en mode semi-continu.

14. Procédé selon l'une quelconque des revendications précédentes, **caractérisé en ce que** l'on utilise, comme éduit, du pseudocumène.

15. Procédé selon l'une quelconque des revendications précédentes, **caractérisé en ce que** l'on emploie la valeur de pH du milieu réactionnel comme grandeur de réglage pour la productivité du biocatalyseur.

# Fig. 1

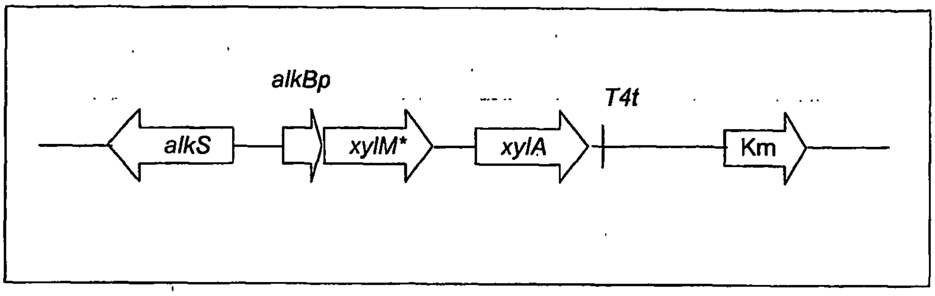


# Fig. 2

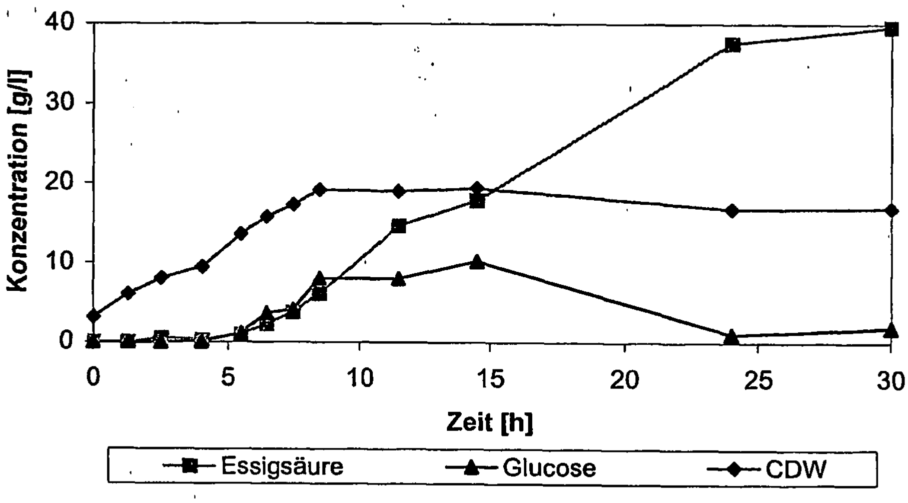


# Fig. 3

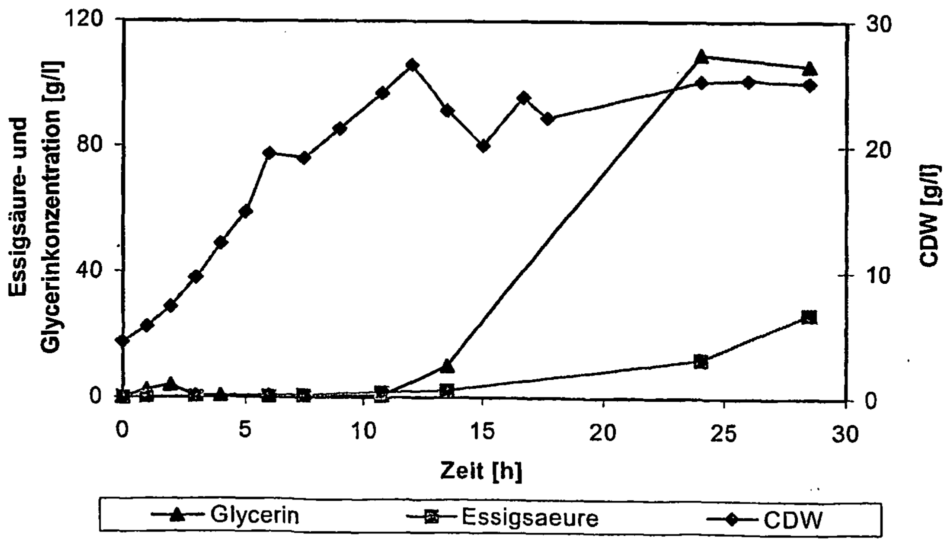

Fig. 4a

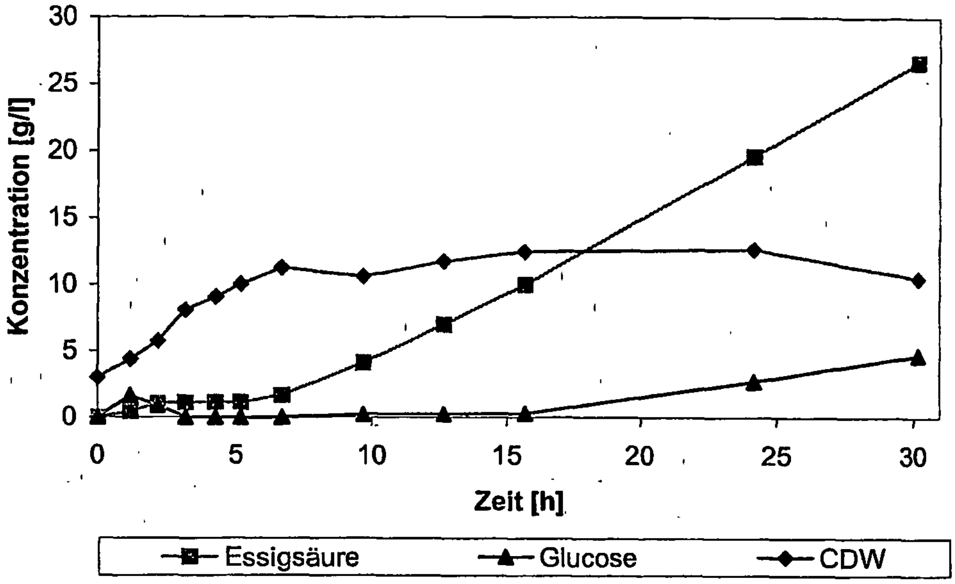
Konzentration [g/l]
30
25
20
15
10
5
0
0
5
10
15
20
25
30
Zeit [h]
Essigsäure
Glucose
CDW

Fig. 4b

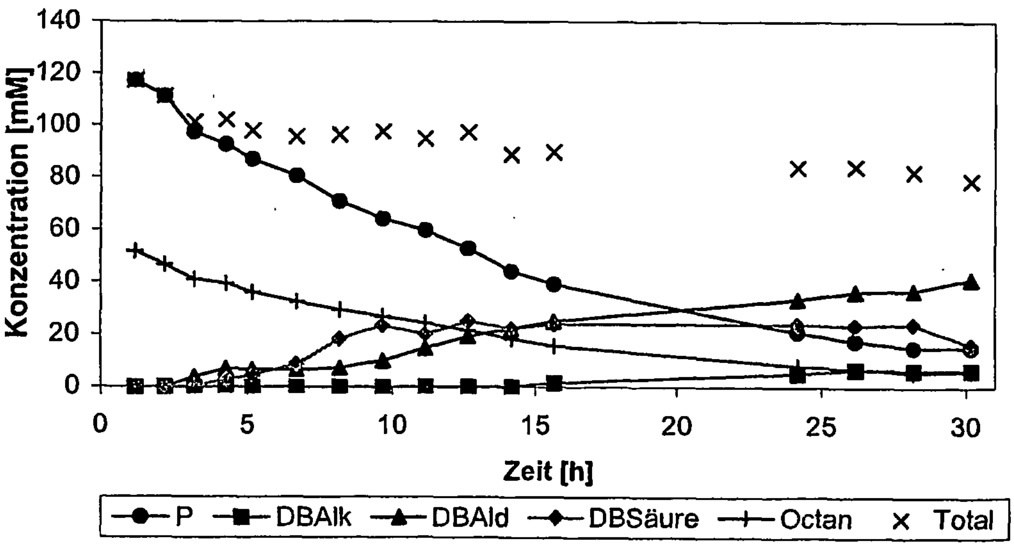
Konzentration [mM]
140
120
100
80
60
40
20
0
0
5
10
15
20
25
30
Zeit [h]
P
DBAlk
DBAld
DBSäure
Octan
Total

Fig. 5

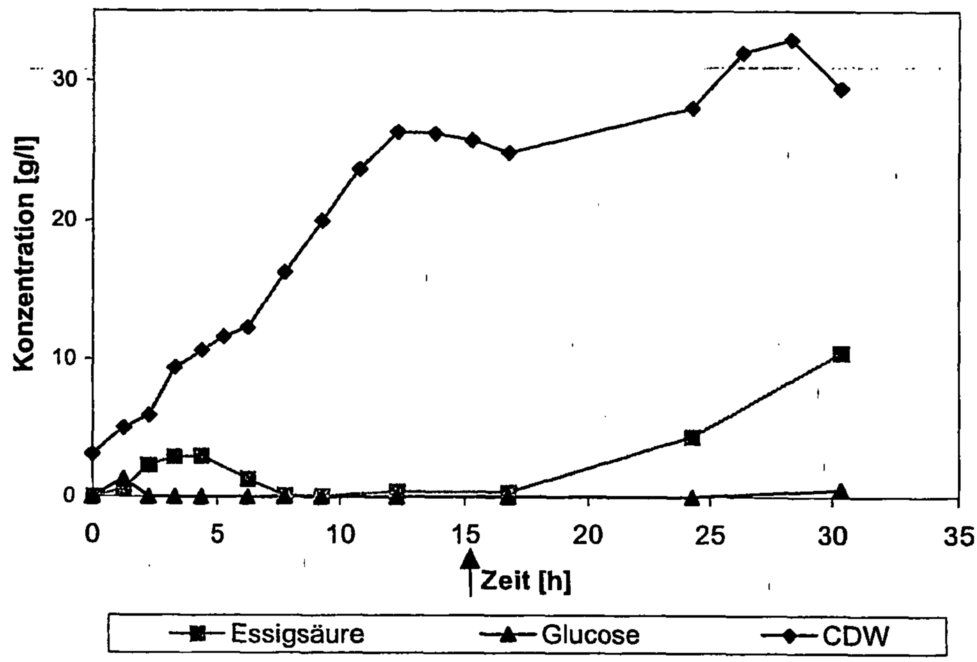
Konzentration [g/l]
30
20
10
0
0
5
10
15
20
25
30
35
Zeit [h]
Essigsäure
Glucose
CDW

Fig. 6

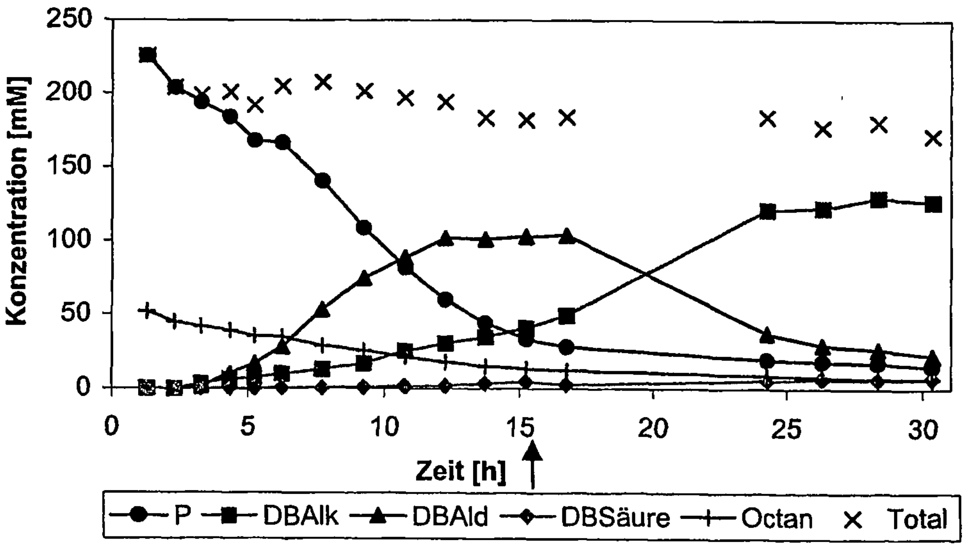
Konzentration [mM]
250
200
150
100
50
0
0
5
10
15
20
25
30
Zeit [h]
P
DBAlk
DBAld
DBSäure
Octan
Total

Fig. 7

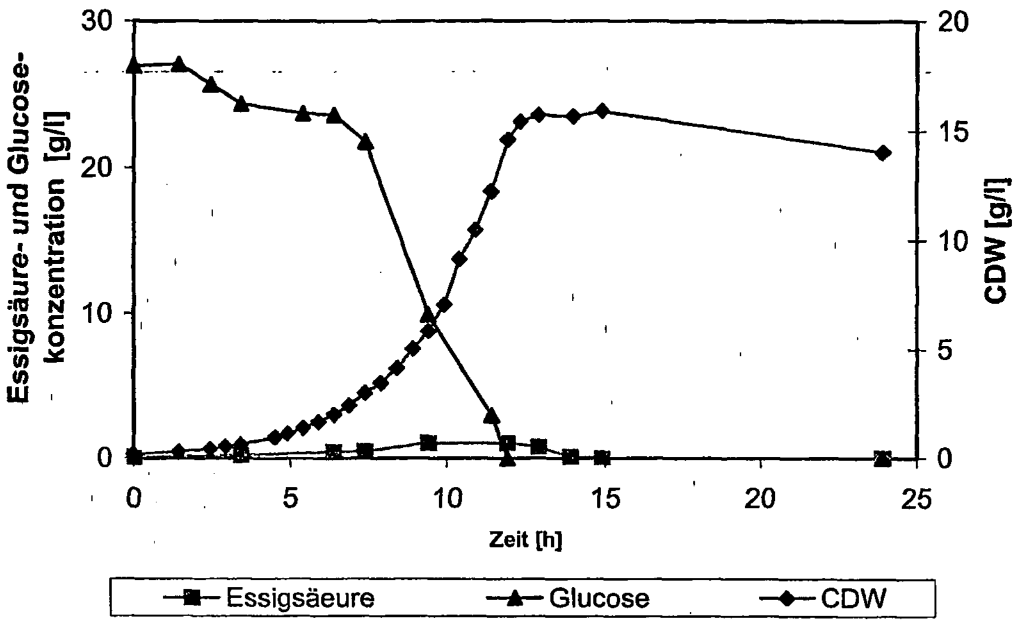
30
20
10
0
Essigsäure- und Glucose-konzentration [g/l]
20
15
10
5
0
CDW [g/l]
0
5
10
15
20
25
Zeit [h]
Essigsäeure
Glucose
CDW

Fig. 8

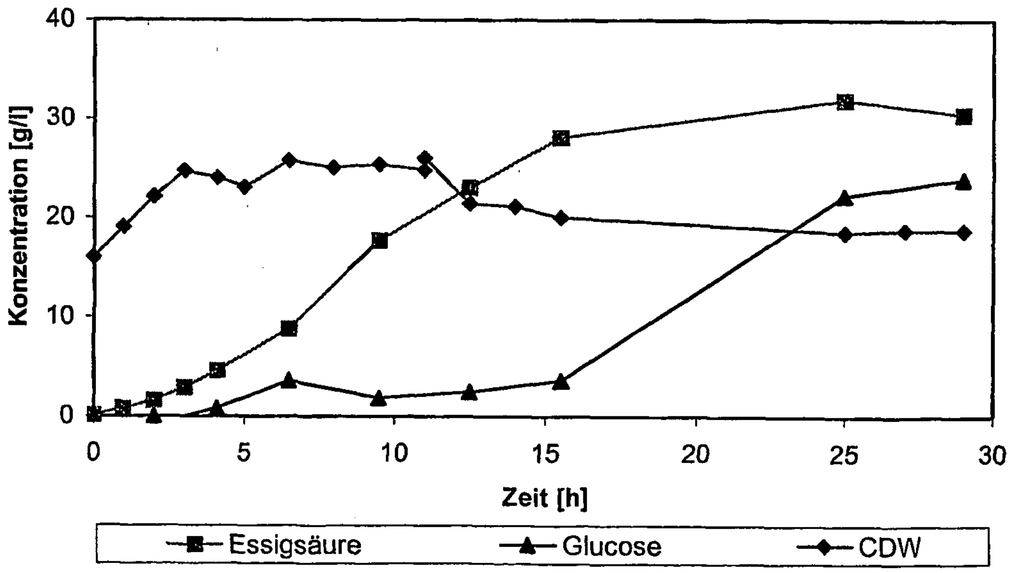
40
30
20
10
0
Konzentration [g/l]
0
5
10
15
20
25
30
Zeit [h]
Essigsäure
Glucose
CDW

Fig. 9

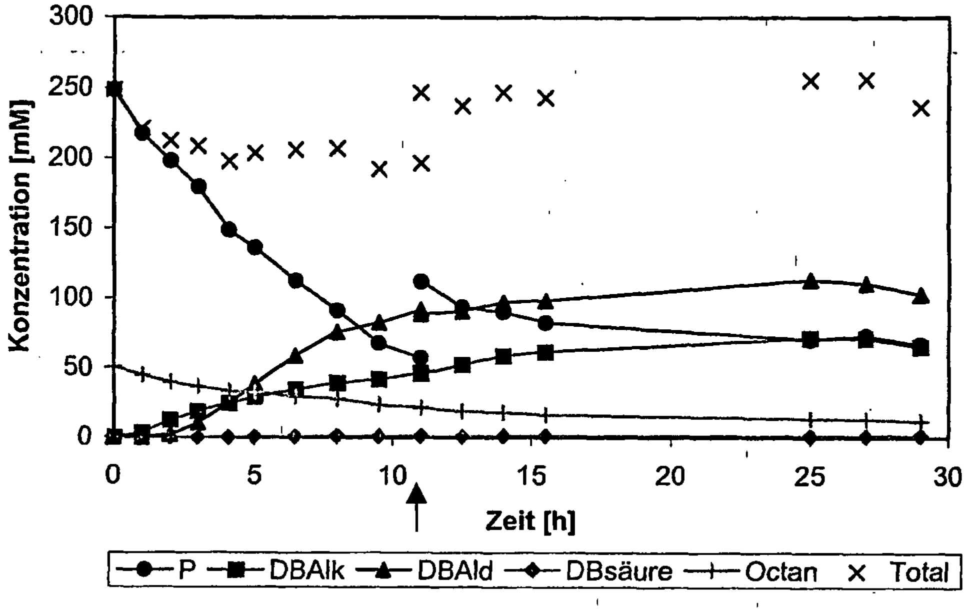
300
250
200
150
100
50
0
Konzentration [mM]
0
5
10
15
20
25
30
Zeit [h]
P
DBAlk
DBAld
DBsäure
Octan
Total

Fig. 10

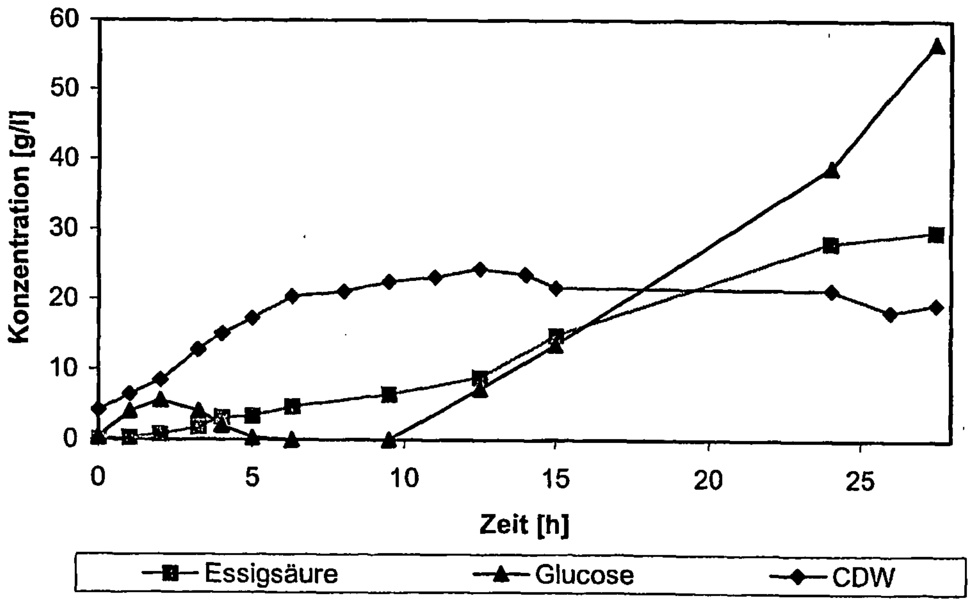
60
50
40
30
20
10
0
Konzentration [g/l]
0
5
10
15
20
25
Zeit [h]
Essigsäure
Glucose
CDW

# Fig. 11

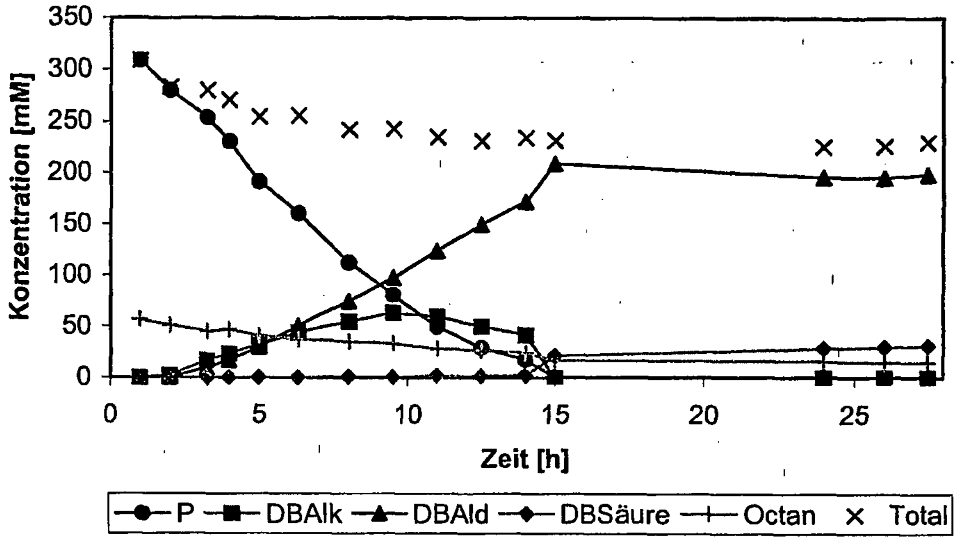
350
300
250
200
150
100
50
0
Konzentration [mM]
0
5
10
15
20
25
Zeit [h]
P
DBAlk
DBAld
DBSäure
Octan
Total

# Fig. 12

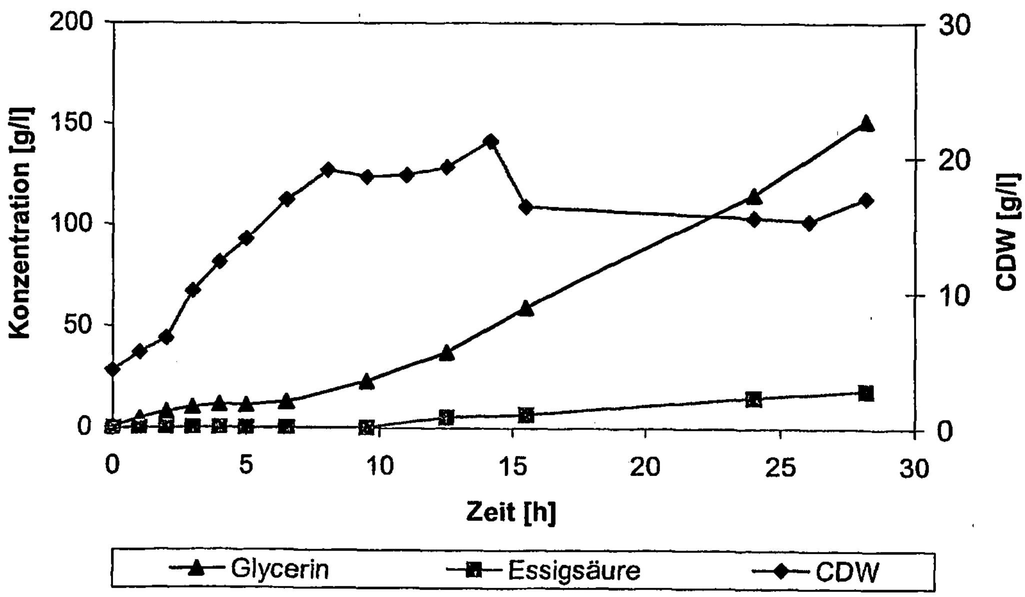
200
150
100
50
0
Konzentration [g/l]
30
20
10
0
CDW [g/l]
0
5
10
15
20
25
30
Zeit [h]
Glycerin
Essigsäure
CDW

Fig. 13

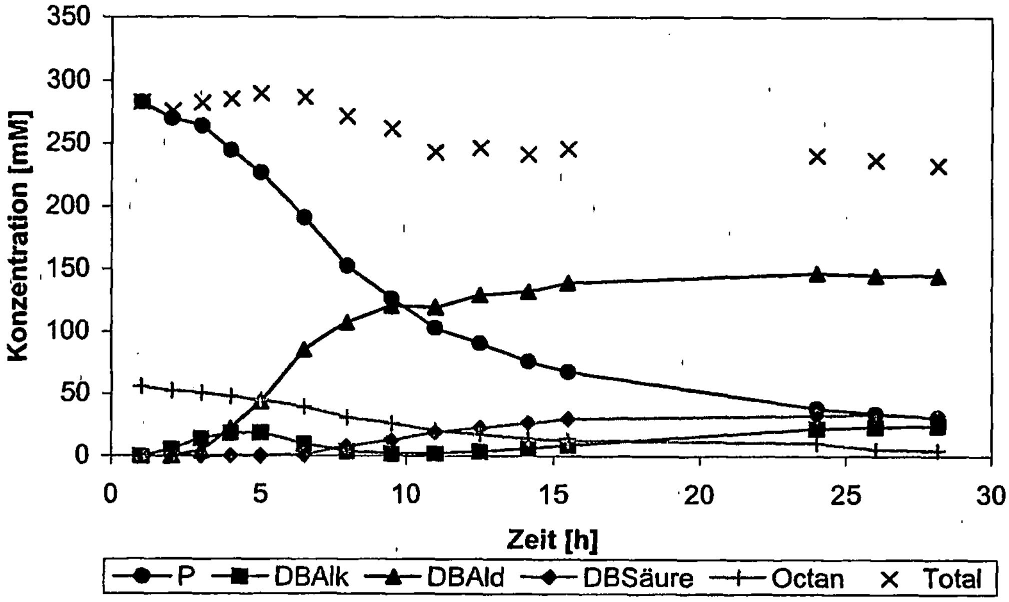
350
300
250
200
150
100
50
0
Konzentration [mM]
0
5
10
15
20
25
30
Zeit [h]
P
DBAlk
DBAld
DBSäure
Octan
Total

Fig. 14

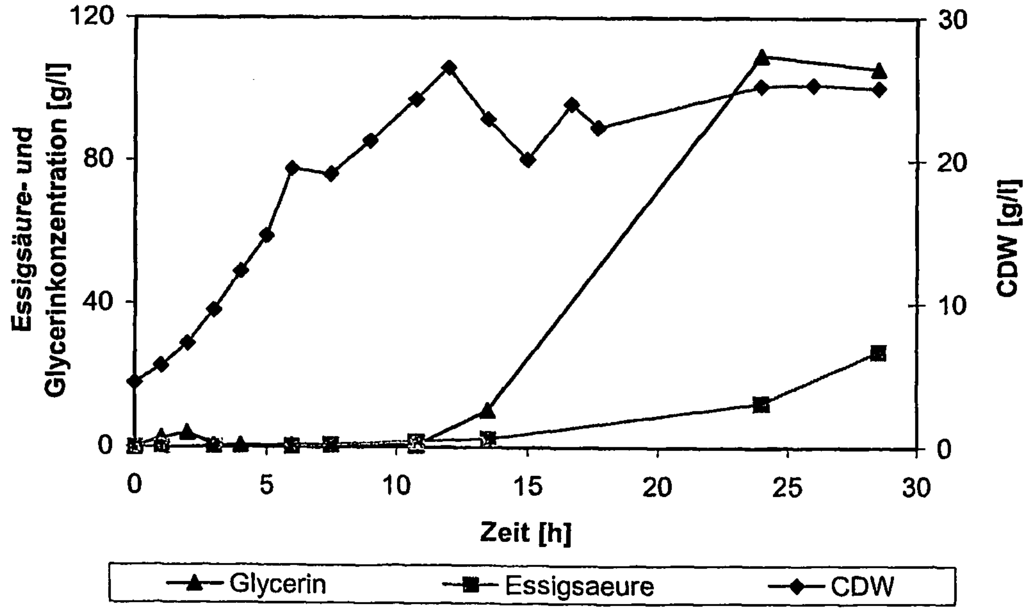
120
80
40
0
Essigsäure- und Glycerinkonzentration [g/l]
30
20
10
0
CDW [g/l]
0
5
10
15
20
25
30
Zeit [h]
Glycerin
Essigsaeure
CDW

Fig. 15

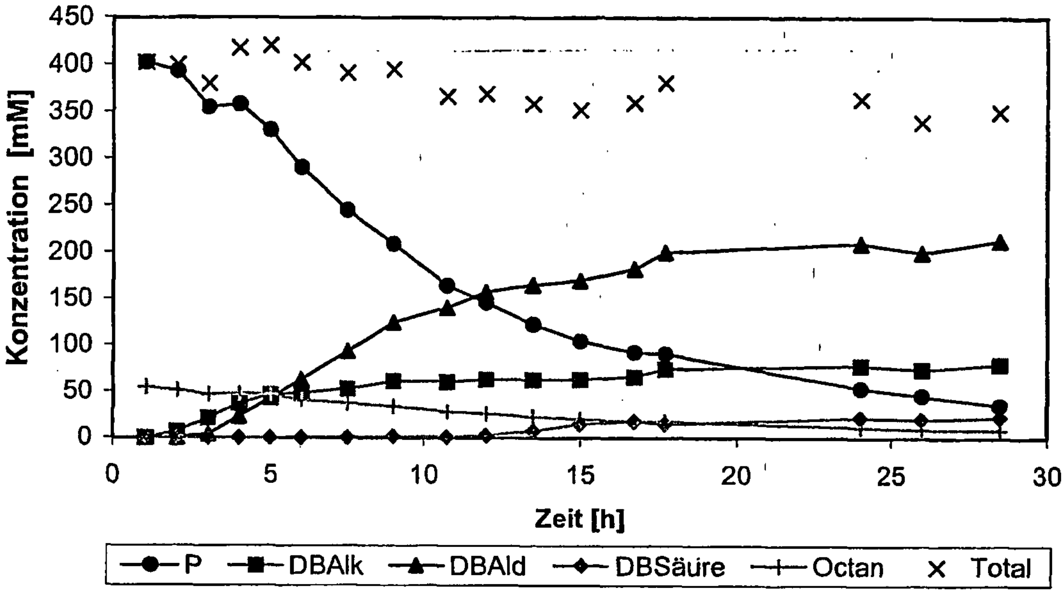
Konzentration [mM]
450
400
350
300
250
200
150
100
50
0
0
5
10
15
20
25
30
Zeit [h]
P
DBAlk
DBAld
DBSäure
Octan
Total

Fig. 16

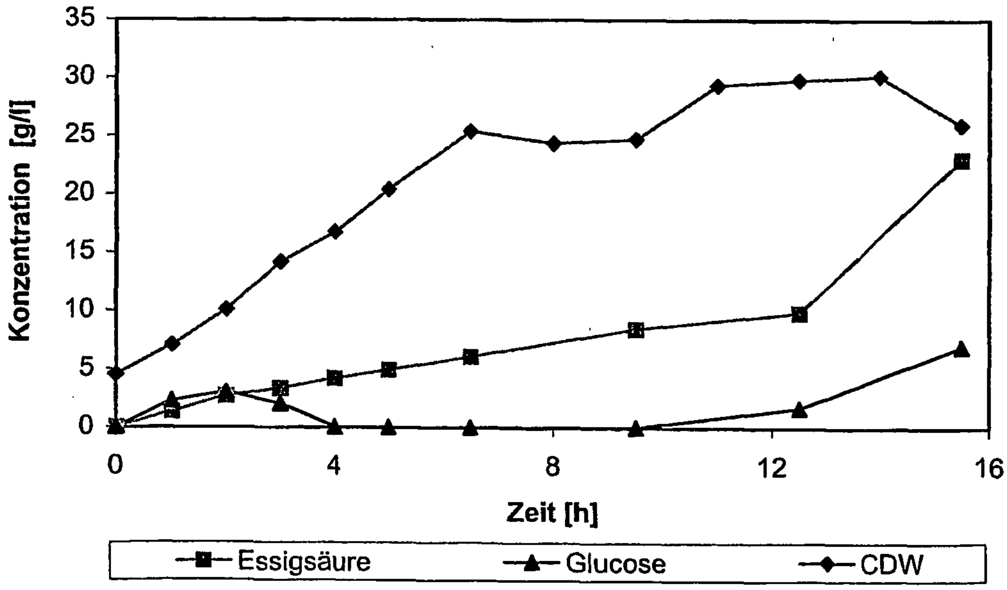
Konzentration [g/l]
35
30
25
20
15
10
5
0
0
4
8
12
16
Zeit [h]
Essigsäure
Glucose
CDW

# Fig. 17

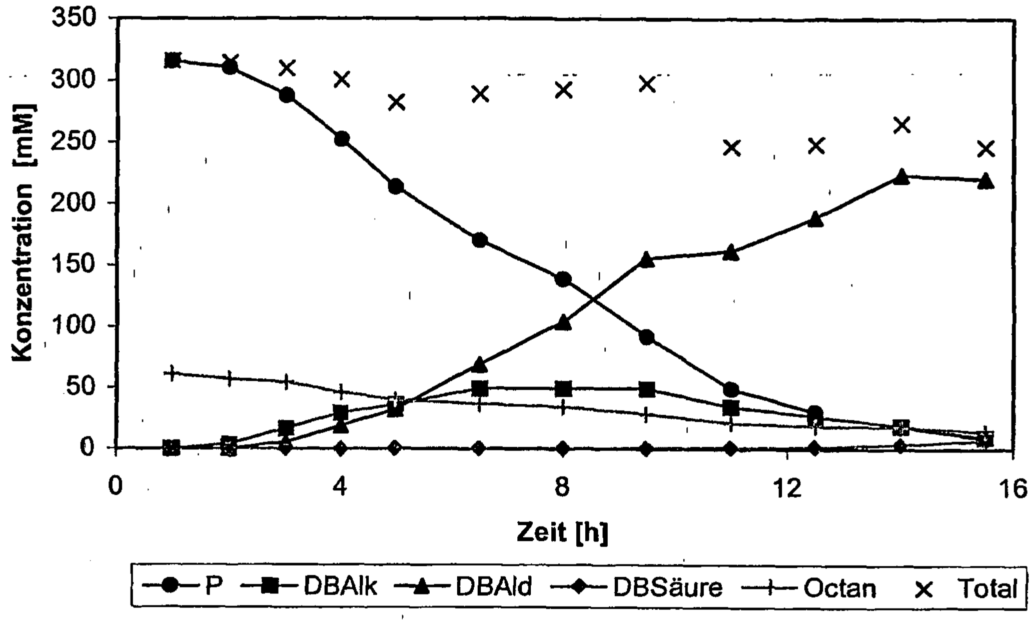
350
300
250
200
150
100
50
0
Konzentration [mM]
0
4
8
12
16
Zeit [h]
P
DBAlk
DBAld
DBSäure
Octan
Total

# Fig. 18

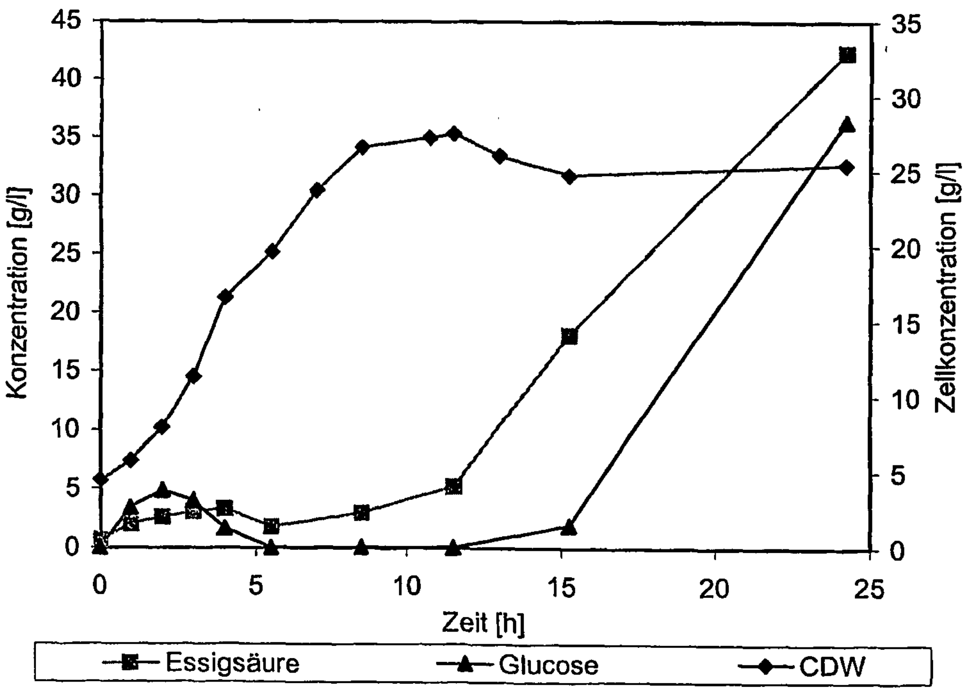
45
40
35
30
25
20
15
10
5
0
Konzentration [g/l]
35
30
25
20
15
10
5
0
Zellkonzentration [g/l]
0
5
10
15
20
25
Zeit [h]
Essigsäure
Glucose
CDW

Fig. 19

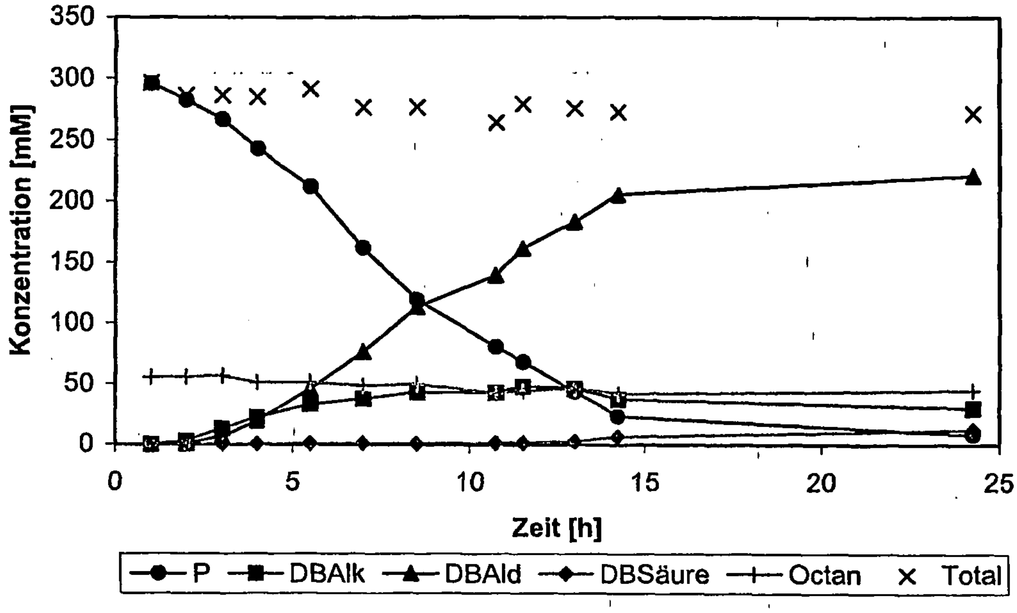
Konzentration [mM]
350
300
250
200
150
100
50
0
0
5
10
15
20
25
Zeit [h]
P
DBAlk
DBAld
DBSäure
Octan
Total

Fig. 20

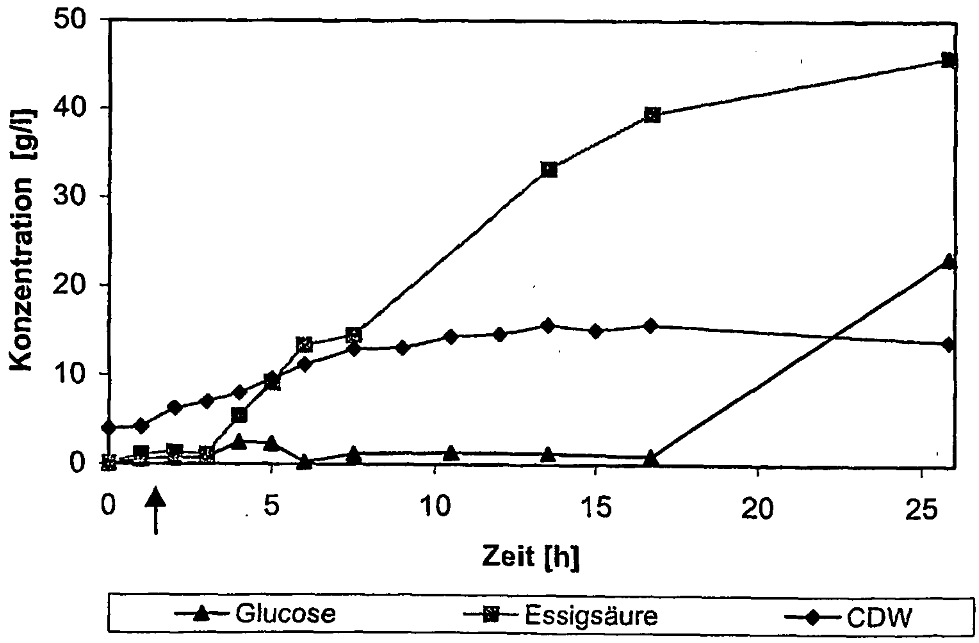
Konzentration [g/l]
50
40
30
20
10
0
0
5
10
15
20
25
Zeit [h]
Glucose
Essigsäure
CDW

Fig. 21

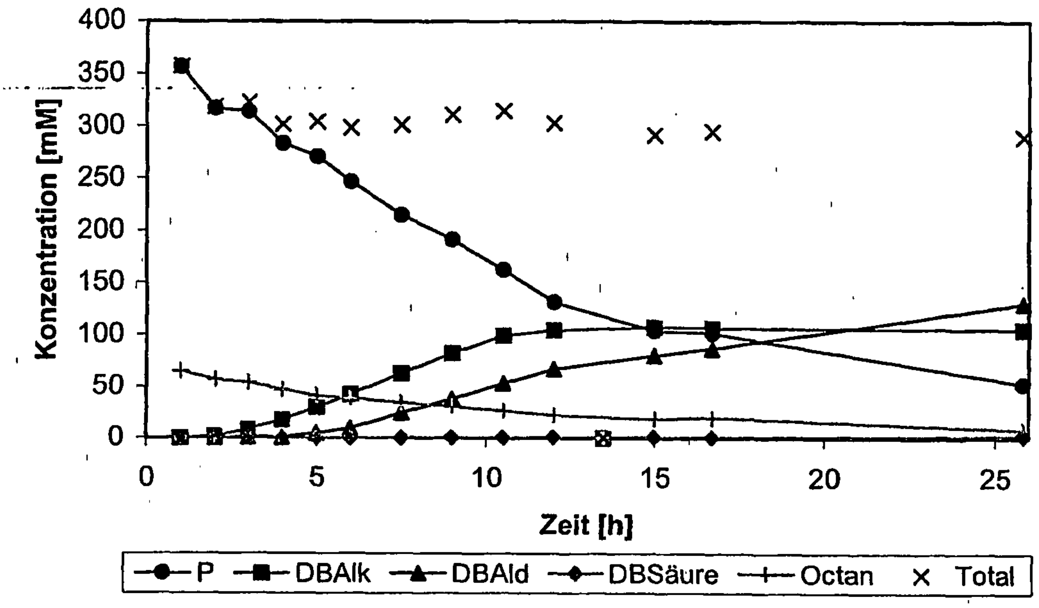
Konzentration [mM]
400
350
300
250
200
150
100
50
0
0
5
10
15
20
25
Zeit [h]
P
DBAlk
DBAld
DBSäure
Octan
Total

Fig. 22

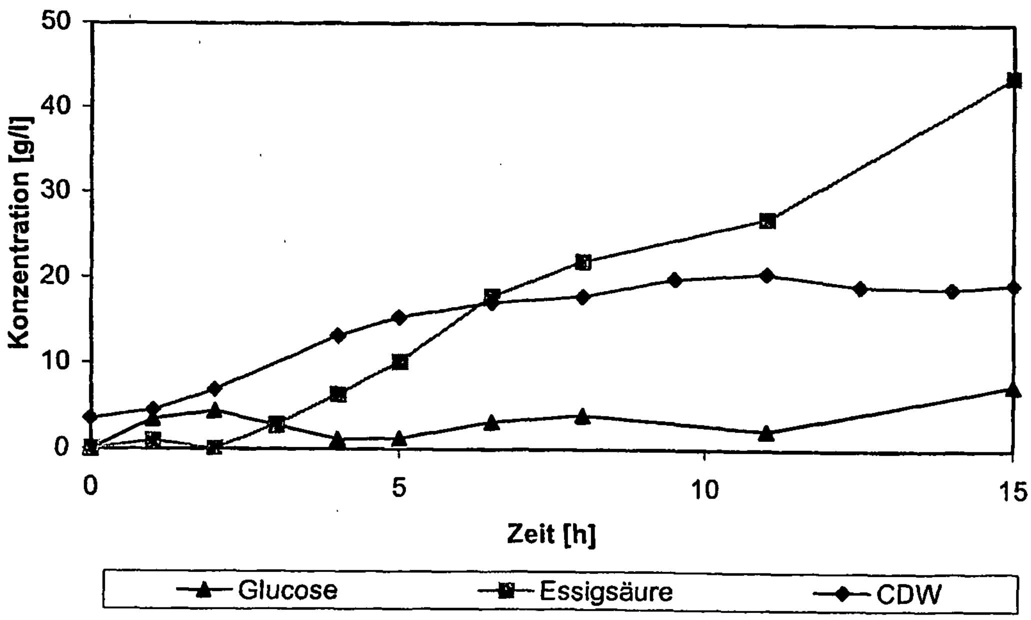
Konzentration [g/l]
50
40
30
20
10
0
0
5
10
15
Zeit [h]
Glucose
Essigsäure
CDW

Fig. 23

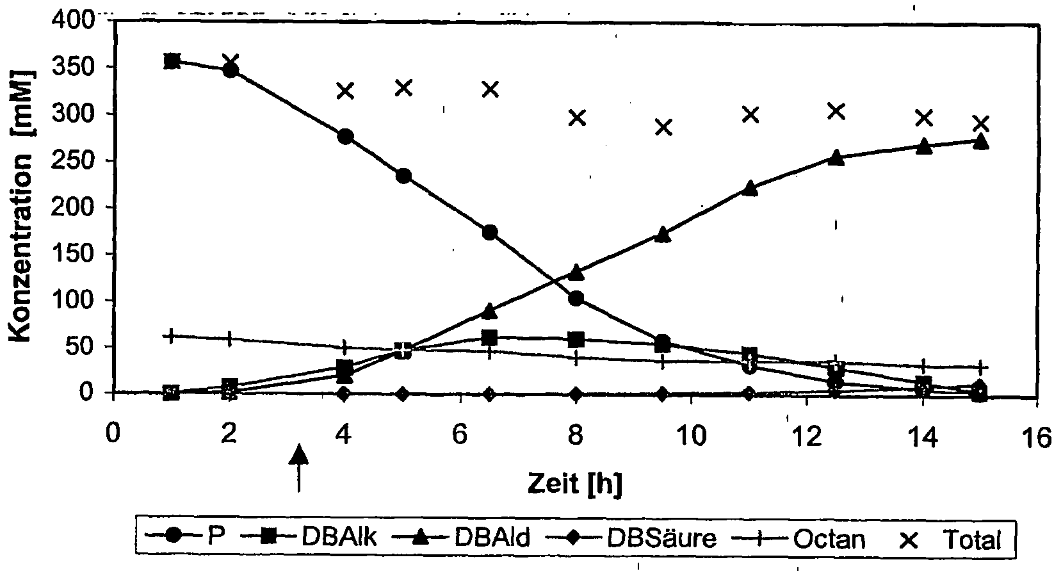
Konzentration [mM]
400
350
300
250
200
150
100
50
0
0
2
4
6
8
10
12
14
16
Zeit [h]
P
DBAlk
DBAld
DBSäure
Octan
Total

Fig. 24

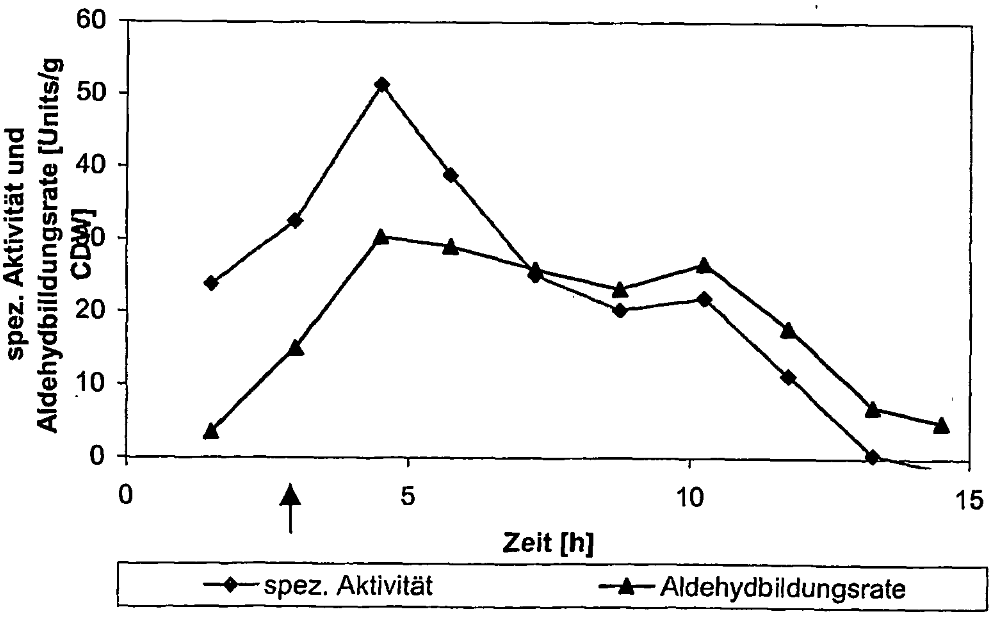
spez. Aktivität und Aldehydbildungsrate [Units/g CDW]
60
50
40
30
20
10
0
0
5
10
15
Zeit [h]
spez. Aktivität
Aldehydbildungsrate

# Fig. 25

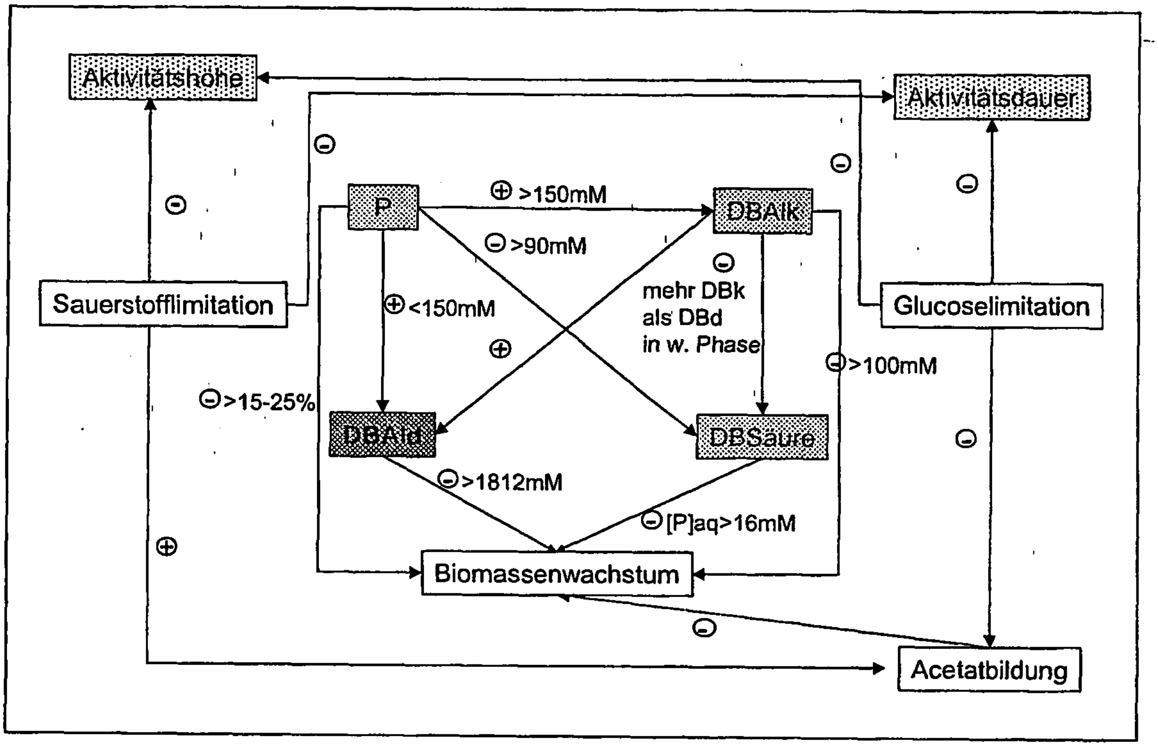
Aktivitätshöhe
Aktivitätsdauer
P
⊕ >150mM
DBAlk
⊖ >90mM
⊖
mehr DBk
als DBd
in w. Phase
⊕<150mM
⊕
Sauerstofflimitation
Glucoselimitation
⊖>15-25%
⊖>100mM
DBAld
DBSäure
⊖>1812mM
⊖[P]aq>16mM
⊕
Biomassenwachstum
⊖
Acetatbildung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19951768 A


### In der Beschreibung aufgeführte Nicht-Patentliteratur


- **F. AUSUBEL et al.** Current Protocols in Molecular Biology. Wiley Interscience, 1997
- **BÜHLER, B. ; A. SCHMID et al.** Xylene monooxygenase catalyzes the multistep oxygenation of toluene and pseudocumene to corresponding alcohols, aldehydes, and acids in Escherichia coli JM101. *The Journal of Biological Chemistry,* 2000, vol. 275 (14), 10085-10092
- **HARAYAMA, S. ; M. KOK et al.** Functional and evolutionary relationships among diverse oxygenases. *Ann. Rev. Microbiol.,* 1992, vol. 46, 565-601
- **HARAYAMA, S. ; R. A. LEPPIK et al.** Gene order of the TOL catabolic plasmid upper pathway operon and oxidation of both toluene and benzyl alcohol by the xylA product. *Journal of Bacteriology,* 1986, vol. 167 (2), 455-461
- **HARAYAMA, S. ; M. REKIK et al.** Characterization of five genes in the upperpathway operon of TOL plasmid pWW0 from Pseudomonas putida and identification of the gene products. *Journal of Bacteriology,* 1989, vol. 171 (9), 5048-5055
- **LEGOY, M. D. ; H. S. KIM et al.** Use of alcohol dehydrogenase for flavor aldehyde production. *Process Biochemistry,* 1985, vol. 20, 145-148
- **MOLINARI, F. ; F. ARAGOZZINI et al.** Continuous production of isovaleraldehyde through extractive bioconversion in a hollow-fiber membrane bioreactor. *Enzyme and Microbial Technology,* 1997, vol. 20, 604-611
- **MOLINARI, F. ; R. GANDOLFI et al.** Biotransformations in two-liquid-phase systems. Production of phenylacetaldehyde by oxidation of 2-phenylethanol with acetic acid bacteria. *Enzyme and Microbial Technology,* 1999, vol. 25, 729-735
- **MOLINARI, F. ; R. VILLA et al.** Aldehyde production by alcohol oxidation with Gluconobacter oxydans. *Applied Microbiology and Biotechnology,* 1995, vol. 43, 989-994
- Production of (S)-styrene oxide with recombinant bacteria. **PANKE, S.** Institute of Biotechnolgy. 1999, 208
- **POUWELS P. H. et al.** Cloning Vectors. Elsevier, 1985
- **RAMOS, J. L. ; S. MARQUÉS et al.** Transcriptional control of the Pseudomonas TOL plasmid catabolic operons is achieved through an interplay of host factors and plasmid-encoded regulators. *Annual Reviews in Microbiology,* 1997, vol. 51, 341-373
- **RIESENBERG, D.** High-cell-density cultivation of Escherichia coli. *Current Opinion in Biotechnology,* 1991, vol. 2, 380-384
- **SAMBROOK, J. ; E. F. FRITSCH et al.** Molecular cloning: a laboratory manual. Cold Spring Harbor Laboratory, 1989
- **SCHMID, A.** Der Metabolismus von ortho-Hydroxybiphenylen. *Pseudomonas,* 1997
- **SIMMONDS, J. ; G. K. ROBINSON.** Novel biotransformations to produce aromatic and heterocyclic aldehydes. *Enzyme and Microbial Technology,* 1997, vol. 21, 367-374
- **SIMMONDS, J. ; G. K. ROBINSON.** Formation of benzaldehyde by Pseudomonas putida ATCC 12633. *Applied Microbiology and Biotechnology,* 1998, vol. 50, 353-358
- **WILLIAMS, P. A. ; L. M. SHAW et al.** xy1UW, two genes at the start of the upper pathway operon of TOL plasmid pWW0, appear to play no essential part in determining its catabolic potential. *Microbiology,* 1997, vol. 143, 101-107
- **WITTCOFF, H. A. ; B. G. REUBEN.** Industrial Organic Chemicals. John Wiley & Sons . Inc, 1996